# FASCICULE DE BREVET EUROPEEN

(11) **EP 2 814 461 B1**
(45) Date de publication et mention de la délivrance du brevet: **24.07.2019**
(21) Numéro de dépôt: 13701849.5
(22) Date de dépôt: 09.01.2013
(51) Int. Cl.: A61K 9/08, A61K 38/26, A61K 38/28, A61K 47/30, A61K 47/42, A61K 9/00, A61P 3/10

(54) **SOLUTION INJECTABLE A PH 7 COMPRENANT AU MOINS UNE INSULINE BASALE DONT LE PI EST COMPRIS ENTRE 5,8 ET 8,5 ET UN CO-POLYAMINOACIDE SUBSTITUE**
INJEKTIONSLÖSUNG MIT EINEM PH-WERT VON 7 MIT WENIGSTENS BASALINSULIN MIT EINEM PI ZWISCHEN 5,8 UND 8,5 SOWIE SUBSTITUIERTER CO-POLY(AMINOSÄURE)
INJECTABLE SOLUTION HAVING A PH OF 7 AND INCLUDING AT LEAST BASAL INSULIN, THE PI OF WHICH IS BETWEEN 5.8 AND 8.5, AND A SUBSTITUTED CO-POLY(AMINO ACID)

(30) Priorité: 09.01.2012 FR 1250224; 09.01.2012 US 201261584623 P; 09.01.2012 FR 1250223; 09.01.2012 US 201261584625 P
(43) Date de publication de la demande: 24.12.2014
(73) Titulaire: Adocia, 69003 Lyon (FR)
(72) Inventeur: SOULA, Olivier, F-69330 Meyzieu (FR)
(74) Mandataire: Tripoz, Inès
(86) Numéro de dépôt international: PCT/FR2013/050043
(87) Numéro de publication internationale: WO 2013/104861

(56) Documents cités:
- EP-A2- 2 387 989
- WO-A1-2004/060968
- WO-A2-2007/141344
- WO-A2-2009/063072
- FR-A1- 2 840 614
- FR-A1- 2 862 536
- HUILLE S ET AL: "Controlled release of insulin from nanoparticles of amphiphilic block copolyamino acid", JOURNAL OF CONTROLLED RELEASE, ELSEVIER, AMSTERDAM, NL, vol. 64, no. 1-3, 14 février 2000 (2000-02-14), pages 319-321, XP001525877, ISSN: 0168-3659 [extrait le 2003-06-20]
- "Moins de variabilité glycémique et meilleurs résultats rapportés avec Lantus® et Apidra® par rapport aux analogues de l'insuline premix", Communiqué de presse sanofi-aventis , 26 juin 2010 (2010-06-26), XP002686280, Extrait de l'Internet: URL:http://www.sanofi.com/Images/13756_201 00626_ada_pro_release_fr.pdf [extrait le 2012-10-31]
- "BYETTA® Approved for Use with Insulin Glargine in the U.S.", Lilly News , 19 octobre 2011 (2011-10-19), XP002686281, Extrait de l'Internet: URL:http://files.shareholder.com/downloads /LLY/2147794056x0x510105/98b5af7e-0826-4bd 4-94c7-2755e0ee0cb8/LLY_News_2011_10_19_Pr oduct.pdf [extrait le 2012-10-31]

## Description

L'invention concerne les thérapies par injection d'insuline(s) pour traiter le diabète.

L'insulinothérapie, ou thérapie du diabète par injection d'insuline, a connu ces dernières années des progrès remarquables grâce notamment à la mise au point de nouvelles insulines offrant une meilleure correction de la glycémie des patients en comparaison de l'insuline humaine et qui permettent de mieux simuler l'activité physiologique du pancréas.

Lorsqu'un diabète de type II est diagnostiqué chez un patient, un traitement graduel est mis en place. Le patient prend en premier lieu des antidiabétiques oraux (OAD) comme la Metformine. Lorsque les OAD seuls ne suffisent plus à réguler le niveau de glucose dans le sang un changement dans le traitement doit être fait et, en fonction des spécificités des patients, différentes associations de traitement peuvent être mises en place. Le patient peut par exemple avoir un traitement à base d'une insuline basale de type glargine ou detemir en complément des OAD, puis ensuite en fonction de l'évolution de la pathologie un traitement à base d'insuline basale et d'insuline prandiale.

Par ailleurs, aujourd'hui, pour assurer la transition des traitements par les OAD, lorsque ceux-ci ne sont plus en mesure de contrôler le niveau de glucose dans le sang, vers un traitement insuline basale/insuline prandiale, l'injection d'analogues de GLP-1 est préconisée.

Les GLP-1 pour Glucagon-Like Peptide-1, sont des peptides insulinotropiques ou incrétines, et appartiennent à la famille des hormones gastro-intestinales (ou Gut Hormones) qui stimulent la sécrétion d'insuline lorsque la glycémie est trop élevée, par exemple après un repas.

Les hormones gastro-intestinales (Gut hormones) sont aussi appelées hormones de satiété. Elles comprennent notamment le GLP-1 (Glucagon like peptide-1) et le GIP (Glucose-dependent insulinotropic peptide), l'oxyntomoduline (un dérivé du proglucagon), le peptide YY, l'amyline, la cholecystokinine, le polypeptide pancréatique (PP), la ghreline et l'entérostatine qui ont des structures peptidiques ou protéiques. Elles stimulent également la secrétion d'insuline, en réponse au glucose et aux acides gras et sont donc à ce titre des candidats potentiels pour le traitement du diabète.

Parmi celles-ci, les GLP-1 sont celles qui ont apporté à ce jour les meilleurs résultats dans le développement de médicaments. Elles ont permis à des patients atteints de diabète de type II de perdre du poids tout en ayant un meilleur contrôle de leur glycémie.

Des analogues ou des dérivés de GLP-1 ont ainsi été développés notamment pour améliorer leur stabilité.

D'autre part, pour couvrir ses besoins journaliers en insuline, un patient diabétique dispose, actuellement, de façon schématisée, de deux types d'insulines ayant des actions complémentaires : les insulines prandiales (ou insulines dites à action rapide) et les insulines basales (ou insulines dites à action lente).

Les insulines prandiales permettent une prise en charge rapide (métabolisation et/ou stockage) du glucose apporté lors des repas et collations. Le patient doit s'injecter une insuline prandiale avant chaque prise alimentaire, soit environ 2 à 3 injections par jour. Les insulines prandiales les plus utilisées sont : l'insuline humaine recombinante, NovoLog® (insuline aspart de NOVO NORDISK), Humalog® (insuline lispro de ELI LILLY) et Apidra® (insuline glulisine de SANOFI-AVENTIS).

Les insulines basales assurent le maintien de l'homéostasie glycémique du patient, en dehors des périodes de prise alimentaire. Elles agissent essentiellement pour bloquer la production endogène de glucose (glucose hépatique). La dose journalière d'insuline basale correspond généralement à 40-50 % des besoins totaux journaliers en insuline. Selon l'insuline basale utilisée, cette dose est dispensée en 1 ou 2 injections, régulièrement réparties au cours de la journée. Les insulines basales les plus utilisées sont Levemir® (insuline detemir de NOVO NORDISK) et Lantus® (insuline glargine de SANOFI-AVENTIS),

On notera pour être exhaustif que la NPH (insuline NPH pour Neutral Protamine Hagedorn ; Humuline NPH®, Insulatard®) est la plus ancienne insuline basale, Cette formulation est le résultat d'une précipitation de l'insuline humaine (anionique à pH neutre) par une protéine cationique, la protamine. Les microcristaux ainsi formés sont dispersés dans une suspension aqueuse et se dissolvent lentement après injection sous-cutanée. Cette dissolution lente assure une libération prolongée de l'insuline. Cependant cette libération n'assure pas une concentration constante d'insuline au cours du temps. Le profil de libération est en forme de cloche et dure seulement entre 12 et 16 heures. Elle est donc injectée deux fois par jour. Cette insuline basale NPH est bien moins performante que les insulines basales modernes, Levemir® et Lantus®. La NPH est une insuline basale à action intermédiaire.

Le principe de la NPH a évolué avec l'apparition des insulines analogues rapides pour donner des produits appelés « Premix » offrant à la fois une action rapide et une action intermédiaire. NovoLog Mix® (NOVO NORDISK) et Humalog Mix® (ELI LILLY) sont des formulations comprenant une insuline analogue rapide, Novolog® et Humalog®, complexée partiellement par la protamine. Ces formulations contiennent ainsi des microcristaux d'insuline analogue dont l'action est dite intermédiaire et une partie d'insuline restée soluble dont l'action est rapide. Ces formulations offrent bien l'avantage d'une insuline rapide mais elles ont aussi le défaut de la NPH, c.-à-d. une durée d'action limitée entre 12 et 16 heures et une insuline libérée en « cloche ». Cependant, ces produits permettent au patient de s'injecter en une seule fois une insuline basale à action intermédiaire avec une insuline prandiale à action rapide. Or nombreux sont les patients soucieux de réduire leur nombre d'injections.

Les insulines basales actuellement commercialisées et actuellement en développement clinique peuvent être classées en fonction de la solution technique qui permet d'obtenir l'action prolongée et à ce jour deux approches sont utilisées.

La première, celle de l'insuline detemir est la liaison à l'albumine *in vivo.* Il s'agit d'un analogue, soluble à pH 7, qui comprend une chaine latérale d'acide gras (tetradecanoyl) fixée à la position B29 qui, *in vivo,* permet à cette insuline de s'associer à l'albumine. Son action prolongée est principalement due à cette affinité pour l'albumine après injection sous-cutanée.

Cependant son profil pharmacocinétique ne permet pas de couvrir une journée, ce qui fait qu'elle est le plus souvent utilisée en deux injections par jour.

D'autres insulines basales solubles à pH 7, comme Degludec® sont actuellement en développement. Degludec® comprend également une chaîne latérale d'acide gras fixée sur l'insuline (hexadecandioyl-γ-L-Glu).

La seconde, celle de l'insuline glargine, est la précipitation à pH physiologique. L'insuline glargine est un analogue de l'insuline humaine obtenu par élongation de la partie C-terminale de la chaine B de l'insuline humaine par deux résidus arginine, et par substitution du résidu d'asparagine A21, par un résidu de glycine (US 5,656,722). L'addition de deux résidus d'arginine a été pensée pour ajuster le pI (point isoélectrique) d'insuline glargine au pH physiologique, et ainsi rendre cet analogue de l'insuline humaine insoluble en milieu physiologique,

Aussi, la substitution de l'A21 a été pensée afin de rendre l'insuline glargine stable à pH acide et pouvoir ainsi la formuler sous forme de solution injectable à pH acide. Lors de l'injection sous-cutanée, le passage de l'insuline glargine d'un pH acide (pH 4-4,5) à un pH physiologique (pH neutre) provoque sa précipitation sous la peau. La redissolution lente des micro-particules d'insuline glargine assure une action lente et prolongée.

L'effet hypoglycémiant de l'insuline glargine est quasi-constant sur une durée de 24 heures ce qui permet à la plupart des patients de se limiter à une seule injection par jour.

L'insuline glargine est considérée aujourd'hui comme la meilleure insuline basale commercialisée.

Cependant le pH nécessairement acide des formulations d'insulines basales, dont le point isoélectrique est compris entre 5,8 et 8,5, de type insuline glargine, peut être un réel inconvénient, car ce pH acide de la formulation d'insuline glargine entraîne parfois chez les patients des douleurs à l'injection et surtout empêche toute formulation avec d'autres protéines et en particulier avec les insulines prandiales car ces dernières ne sont pas stables à pH acide. L'impossibilité de formuler une insuline prandiale, à pH acide, tient au fait qu'une insuline prandiale subit, dans ces conditions, une réaction secondaire de déamidation en position A21 ; ce qui ne permet pas de répondre à l'exigence de la Pharmacopée US, à savoir moins de 5 % de produits secondaires après 4 semaines à 30°C.

Ainsi, personne n'a à ce jour cherché à solubiliser ces insulines basales, de type insuline glargine dont le point isoélectrique est compris entre 5,8 et 8,5, à pH neutre tout en maintenant une différence de solubilité entre le milieu *in-vitro* (le contenant) et le milieu *in-vivo* (sous la peau), indépendamment du pH.

De l'analyse des compositions décrites dans la littérature et les brevets, il apparait que l'insolubilité à pH 7 des insulines basales, du type insuline glargine, est un prérequis pour avoir une action lente.

En effet, le principe de fonctionnement des insulines basales de type insuline glargine, dont le point isoélectrique est compris entre 5,8 et 8,5, est qu'elles sont solubles à pH acide et précipitent à pH physiologique. Ceci détourne l'homme de l'art de toute solution dans laquelle l'insuline de type insuline glargine serait solubilisée à pH 6-8 tout en gardant sa propriété essentielle qui est de précipiter en milieu sous-cutané.

De plus, ce pH acide des formulations des insulines basales, dont le point isoélectrique est compris entre 5,8 et 8,5, de type insuline glargine, empêche même toute combinaison extemporanée avec les insulines prandiales à pH neutre.

En effet, une étude clinique récente, présentée au 69th Scientific Sessions of the American Diabetes Association, New Orleans, Louisiana, 5-9 Juin 2009, a permis de vérifier cette limitation d'usage d'insuline glargine. Une dose d'insuline glargine et une dose d'insuline prandiale (en l'occurrence, l'insuline lispro) ont été mélangées juste avant injection (E. Cengiz et al., 2010 ; Diabetes care - 33(5) : 1009-12). Cette expérience a permis de mettre en évidence un retard significatif dans les profils pharmacocinétique et pharmacodynamique de l'insuline prandiale, pouvant donner lieu à des hyperglycémies postprandiales et à des hypoglycémies nocturnes, Cette étude confirme bien l'incompatibilité de l'insuline glargine avec les insulines à action rapide actuellement commercialisées.

D'ailleurs la notice d'utilisation de Lantus®, le produit commercial à base d'insuline glargine de la société SANOFI-AVENTIS, indique explicitement aux utilisateurs de ne pas réaliser de mélange avec une solution d'insuline prandiale, quelle qu'elle soit, en raison du risque sérieux de modifier la pharmacocinétique et la pharmacodynamie de l'insuline glargine et/ou de l'insuline prandiale mélangée.

Pourtant d'un point de vue thérapeutique, il a été démontré, tel qu'illustré ci-après, que les traitements associant soit une insuline glargine et une insuline prandiale, soit une insuline glargine et un analogue de GLP-1, présentent un réel intérêt.

S'agissant de l'association d'une insuline glargine et d'une insuline prandiale, des études cliniques rendues publiques lors des 70èmes séances scientifiques annuelles de *l'American Diabetes Association* (ADA) de 2010, abstract 2163-PO et abstract numéro 0001-LB, en particulier celles menées par la société SANOFI-AVENTIS, ont montré que les traitements qui associent Lantus®, insuline glargine, et une insuline prandiale sont bien plus efficaces que les traitements à base de produits du type « Premix », Novolog Mix® ou Humalog Mix®.

S'agissant de l'association d'une insuline glargine et d'un analogue de GLP-1, la FDA (Food and Drug Administration, Agence américaine du médicament) a approuvé, en octobre 2011, l'injection d'exenatide (Byetta®, AMYLIN PHARMACEUTICALS, Inc et ELI LILLY and Company) en tant que thérapie complémentaire à l'insuline glargine pour les patients atteints de diabète de type II qui ne sont pas en mesure d'atteindre un contrôle de leur glycémie avec l'analogue d'insuline basale seul.

Or, du fait que le principe même, exposé ci-dessus, des insulines basales dont le point isoélectrique est compris entre 5,8 et 8,5, est qu'elles sont solubles à pH acide et précipitent à pH physiologique, toutes les solutions proposées pour les combiner à d'autres produits comme les insulines prandiales ou des analogues ou dérivés de GLP-1 sont basées sur des essais de solubilisation des insulines prandiales ou des analogues ou dérivés de GLP-1 à pH acide, voir par exemple WO2007/121256, WO2009/021955, WO2011/144673, WO2011/147980 ou encore WO2009/063072.

Par exemple, s'agissant des combinaisons d'insuline glargine et d'insuline rapide, la société BIODEL, a décrit notamment dans la demande de brevet US 7,718,609 des compositions comprenant une insuline basale et une insuline prandiale à un pH compris entre 3,0 et 4,2 en présence d'un agent chélatant et de polyacides. Ce brevet enseigne comment rendre compatible une insuline prandiale à pH acide en présence de l'insuline glargine. Il n'enseigne pas comment préparer une combinaison d'insuline de type insuline glargine et d'une insuline prandiale à pH neutre.

Également à titre d'exemple, s'agissant de la solubilisation de l'insuline glargine à pH neutre et des combinaisons avec un analogue de GLP-1, on citera la demande de brevet WO2011/144676 publiée le 24 novembre 2011, au nom de SANOFI-AVENTIS qui décrit des formulations à pH 9,5 de glargine avec la cyclodextrine SVE4-β-CYD dans lesquelles la solubilité de glargine est améliorée de 0,75 mM à 1,25 mM. Cette demande mentionne également des compositions comprenant en outre un GLP-1, bien que non-exemplifliées. L'effet solubilisant à pH 7,4 dans un tampon phosphate est mentionné. Ces résultats de solubilisation à pH 7,4 sont décrits dans la publication intitulée « Effect of sulfobutyl ether-β-cyclodextrin on bioavailability of insulin glargine and blood glucose level after subcutaneous injection to rats » (International Journal of Pharmaceutics, 419 (2011), 71-76) à la figure 3A. La sulfobutyl ether-β-cyclodextrine améliore la solubilité de l'insuline glargine à pH 7,4 de 5 µM à 8 µM ce qui ne présente pas d'intérêt thérapeutique, dans la mesure où la concentration commerciale de l'insuline glargine est de 600 µM (100 UI/mL). Le problème n'a ainsi pas été résolu de façon satisfaisante par l'invention décrite dans cette demande de brevet.

A notre connaissance, il n'a donc jamais été décrit de formulation, stable à pH physiologique, comprenant une insuline basale dont le point isoélectrique est compris entre 5,8 et 8,5 seule ou en association avec une insuline prandiale et/ou une hormone gastro-intestinale, dans laquelle la solubilité de l'insuline est suffisante pour un traitement thérapeutique.

La présente invention, en résolvant ce problème de solubilité à pH compris entre 6,6 et 7,8 permet :
- de proposer une composition injectable, destinée au traitement du diabète, comprenant une insuline basale, dont le point isoélectrique est compris entre 5,8 et 8,5, sous la forme d'une solution homogène à pH compris entre 6,6 et 7,8, tout en conservant son activité biologique et son profil d'action
- de proposer une composition injectable sous la forme d'une solution homogène à pH compris entre 6,6 et 7,8 comprenant en outre une combinaison d'une insuline basale, dont le point isoélectrique est compris entre 5,8 et 8,5, et d' une insuline prandiale sans modification du profil d'activité de l'insuline prandiale soluble à pH 6-8 et instable à pH acide tout en maintenant le profil d'action lente propre à l'insuline basale
- de proposer une composition injectable sous la forme d'une solution homogène à pH compris entre 6,6 et 7,8 comprenant en outre une combinaison d'une insuline basale, dont le point isoélectrique est compris entre 5,8 et 8,5, et d'un dérivé ou un analogue d'une hormone gastro intestinale comme le GLP-1 ou « glucagon like peptide-1 »,

- de diminuer le nombre d'injections dans le cadre du traitement du diabète,
- aux dites compositions d'être conformes aux exigences des Pharmacopées américaines et européennes.

De façon surprenante, les compositions selon l'invention permettent de solubiliser à un pH compris entre 6,6 et 7,8 une insuline basale dont le point isoélectrique est compris entre 5,8 et 8,5.

De façon surprenante les compositions selon l'invention permettent un maintien de la durée de l'activité hypoglycémiante de l'insuline basale dont le point isoélectrique est compris entre 5,8 et 8,5 malgré sa solubilisation à un pH compris entre 6,6 et 7,8 avant injection. Cette propriété remarquable provient du fait que l'insuline de type insuline glargine solubilisée à un pH compris entre 6,6 et 7,8 dans la composition de l'invention précipite en milieu sous-cutané par un changement de composition du milieu. L'élément déclenchant la précipitation de l'insuline de type insuline glargine n'est plus la modification de pH mais une modification de la composition de l'environnement lors du passage de la composition pharmaceutique du contenant au milieu physiologique. De façon surprenante, dans les combinaisons de l'insuline de type insuline glargine avec une insuline prandiale, objets de l'invention, l'action rapide de l'insuline prandiale est préservée malgré la précipitation de l'insuline de type insuline glargine en milieu sous-cutané.

La solution selon l'invention permettant de solubiliser l'insuline basale, dont le point isoélectrique est compris entre 5,8 et 8,5, à un pH compris entre 6,6 et 7,8, préserve son activité biologique.

Dans les combinaisons de l'insuline de type insuline glargine avec une insuline prandiale, objets de l'invention, l'action rapide de l'insuline prandiale est préservée malgré la précipitation de l'insuline de type insuline glargine en milieu sous-cutané. De plus, la présence de l'insuline prandiale ne modifie pas la solubilité de l'insuline basale à un pH compris entre 6,6 et 7,8 et ne modifie également pas les propriétés de précipitation de l'insuline basale.

L'invention concerne une composition sous forme d'une solution aqueuse injectable, dont le pH est compris entre 6,0 et 8,0, comprenant au moins :
a) une insuline basale dont le point isoélectrique pI est compris entre 5,8 et 8,5 ;
b) un co-polyaminoacide porteur de charges carboxylates et substitué par des groupements hydrophobes, choisi parmi les co-polyaminoacides de formule I : 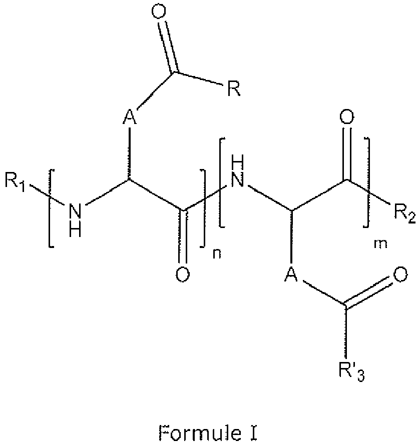 dans laquelle,
   - A représente, indépendamment, soit un groupe -CH₂- (unité aspartique) soit un groupe -CH₂-CH₂- (unité glutamique),
   - R₁ est un radical choisi dans le groupe constitué par un H, un groupe acyle linéaire en C2 à C10, un groupe acyle ramifié en C3 à C10, un benzyle, une unité « acide aminé » terminale et un pyroglutamate,
   - R₂ est un radical -NR'R", R' et R" identiques ou différents étant choisis dans le groupe constitué par H, les alkyles linéaires ou ramifiés ou cycliques en C2 à C30, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S,
   - R'₃ est un radical choisi dans le groupe constitué par les radicaux de formules -OR₃, II ou II': 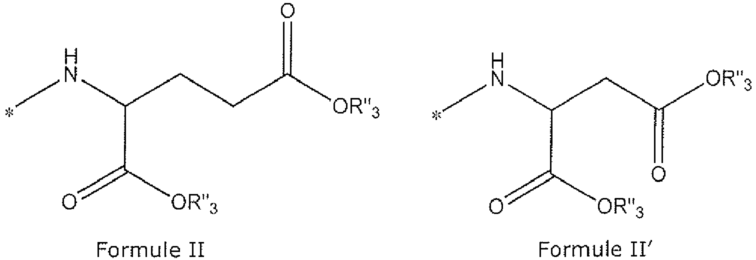 dans lesquelles * indique le site de rattachement au co-polyaminoacide
      - R₃ et R"₃, identiques ou différents, représentent un H ou une entité cationique choisie dans le groupe comprenant les cations métalliques,
   - -R est un radical choisi dans le groupe constitué par un radical en C8 à C30 linéaire ou ramifié, saturé ou insaturé, pouvant comporter des hétéroatomes ou un radical en C8 à C30 pouvant former des cycles carbonés ou pouvant comporter des hétéroatomes saturés, insaturés et/ou aromatiques, lesdits cycles pouvant être ortho-condensés ou péri-condensés, ou un radical de formule III ou III' tel que défini ci-dessous : 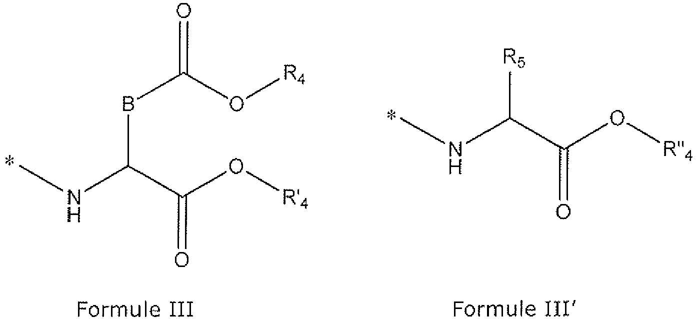 dans lesquelles, * indique le site de rattachement au co-polyaminoacide, et
      - R₄ et R'₄, identiques ou différents représentent un H, une entité cationique choisie dans le groupe comprenant les cations métalliques, un radical R"₄ ou un radical R"'₄, et au moins un des R₄ et R'₄ est égal à R"₄.
         ∘ R"₄ représente un radical en C8 à C30 linéaire ou ramifié, saturé ou insaturé, pouvant comporter des hétéroatomes ou un radical en C8 à C30 pouvant former des cycles carbonés ou pouvant comporter des hétéroatomes saturés, insaturés et/ou aromatiques, lesdits cycles pouvant être ortho-condensés ou péri-condensés,
         ∘ R"'₄ représente un radical en C1 à C7 linéaire ou ramifié, saturé ou insaturé, pouvant comporter des hétéroatomes ou un radical en C1 à C7 pouvant former des cycles carbonés ou pouvant comporter des hétéroatomes saturés, insaturés et/ou aromatiques, lesdits cycles pouvant être ortho-condensés ou péri-condensés, et
      - B représente, indépendamment soit un groupe -CH₂- (unité aspartique) soit un groupe -CH₂-CH₂- (unité glutamique),
      - R₅ est un radical choisi dans le groupe constitué par un H, un alkyle linéaire ou ramifié en C1 à C4 ou un groupement benzyle,
   - n/(n+m) est défini comme le taux de greffage molaire en radical hydrophobe des unités monomériques et est compris entre 1 et 50 % molaire,
   - n + m représente le degré de polymérisation du co-polyaminoacide, c'est-à-dire le nombre moyen d'unités monomériques par chaîne de co-polyaminoacide et 5 ≤ n + m ≤ 1000.

Le co-polyaminoacide, est un co-polyaminoacide statistique.

Les radicaux R'₃, R₃, R"₃, R, R₄, R'₄, R"₄, R'"₄, R', R", R₅ et les groupes A et B, étant chacun indépendamment identiques ou différents d'une unité monomérique à l'autre.

Dans un mode de réalisation la composition selon l'invention est caractérisée en ce que les co-polyaminoacides de formule I peuvent en outre comprendre des unités monomériques de formule VI et/ou VI' :

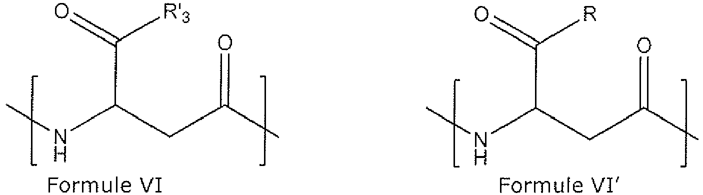

Dans un mode de réalisation, le co-polyaminoacide porteur de charges carboxylates et substitué par des groupements hydrophobes est choisi parmi les co-polyaminoacides de formule IV : 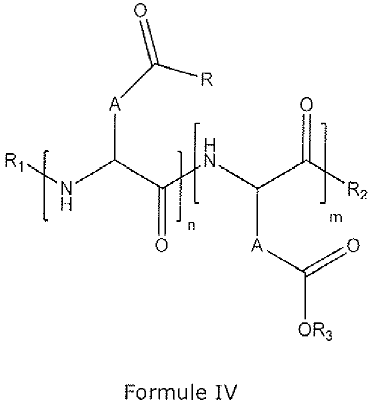 dans laquelle,
- les radicaux ou groupes A, R_{1,} R_{2,} R, et R₃ sont définis comme dans la formule I.

Le co-polyaminoacide, est un co-polyaminoacide statistique.

Les radicaux R'₃, R₃, R"₃, R, R₄, R'₄, R"₄, R"'₄, R', R", R₅ et les groupes A et B, étant chacun indépendamment identiques ou différents d'une unité monomérique à l'autre.

Dans un mode de réalisation, le co-polyaminoacide porteur de charges carboxylates et substitué par des groupements hydrophobes est choisi parmi les co-polyaminoacides de formule IV : 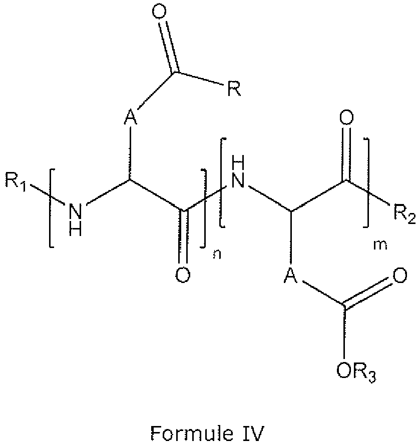 dans laquelle,
- A représente, indépendamment un groupe -CH₂- (unité aspartique) ou un groupe -CH₂-CH₂- (unité glutamique),
- R₁ est choisi dans le groupe constitué par un H, un groupe acyle linéaire en C2 à C10, un groupe acyle ramifié en C3 à C10, un benzyle, une unité « acide aminé » terminale et un pyroglutamate,
- R₂ est un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble des cycles carbonés saturés, insaturés et/ou aromatiques et/ou pouvant comporter des hétéroatomes, choisis dans le groupe constitué par O, N et S,
- les groupes R₃ identiques ou différents sont choisis dans le groupe constitué par un H ou une entité cationique choisie dans le groupe comprenant les cations métalliques,
- les groupes R représentent chacun indépendamment les uns des autres un radical choisi parmi les radicaux de formule générale V ou V' : 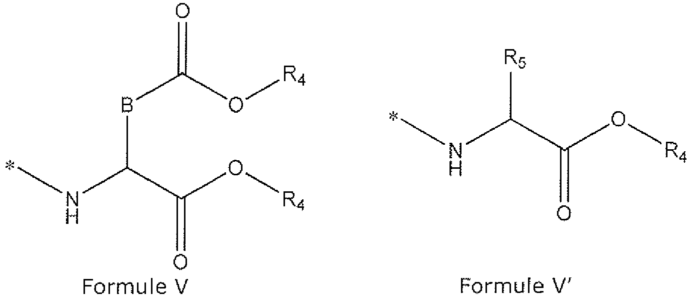 dans lesquelles, * indique le site de rattachement au co-polyaminoacide, et,
   - R₄ représente un radical en C8 à C30 linéaire ou ramifié, saturé ou insaturé, pouvant comporter des hétéroatomes ou un radical en C8 à C30 pouvant former des cycles carbonés ou pouvant comporter des hétéroatomes saturés, insaturés et/ou aromatiques, lesdits cycles pouvant être ortho-condensés ou péri-condensés,
   - B représente, indépendamment un groupe -CH₂- (unité aspartique) ou un groupe -CH₂-CH₂- (unité glutamique),
   - R₅ représente indépendamment un H, un alkyle linéaire ou ramifié en C1 à C4 ou un groupement benzyle, ou
- R est un radical en C8 à C30 linéaire ou ramifié, saturé ou insaturé, pouvant comporter des hétéroatomes ou un radical en C8 à C30 pouvant former des cycles carbonés ou pouvant comporter des hétéroatomes saturés, insaturés et/ou aromatiques, lesdits cycles pouvant être ortho-condensés ou péri-condensés,
- n/(n+m) est défini comme le taux de greffage molaire en radical hydrophobe des unités monomériques et est compris entre 1 et 50 % molaire,
- n + m représente le degré de polymérisation du co-polyaminoacide, c'est-à-dire le nombre moyen d'unités monomériques par chaîne de co-polyaminoacide et 5 ≤ n + m ≤ 1000.

Le co-polyaminoacide, est un co-polyaminoacide statistique.

Les radicaux R'₃, R₃, R"₃, R, R₄, R'₄, R"₄, R"'₄, R', R", R₅ et les groupes A et B, étant chacun indépendamment identiques ou différents d'une unité monomérique à l'autre.

Dans un mode de réalisation, le co-polyaminoacide porteur de charges carboxylates et substitué par des groupements hydrophobes est choisi parmi les co-polyaminoacides de formule IV : 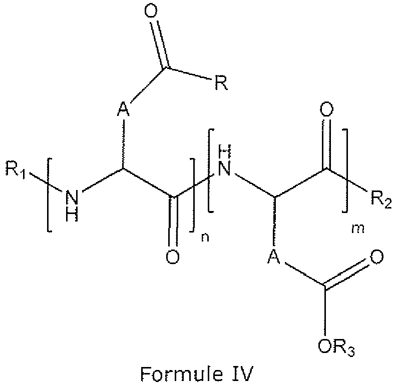 dans laquelle,
- A, représente, indépendamment un groupe -CH₂- (unité aspartique) ou un groupe -CH₂-CH₂- (unité glutamique),
- R₁ est choisi dans le groupe constitué par un H, un groupe acyle linéaire en C2 à C10, un groupe acyle ramifié en C3 à C10, un benzyle, une unité « acide aminé » terminale et un pyroglutamate,
- R₂ est choisi dans le groupe constitué par :
   - un radical amino secondaire -NR'R", R' et R" identiques ou différents étant choisis dans le groupe constitué par les alkyles linéaires ou ramifiés ou cycliques en C2 à C10, le benzyle et lesdits R' et R" alkyles pouvant former ensemble des cycles carbonés saturés, insaturés et/ou aromatiques et/ou pouvant comporter des hétéroatomes, choisis dans le groupe constitué par O, N et S,
- les groupes R₃ identiques ou différents sont choisis dans le groupe constitué par un H ou une entité cationique choisie dans le groupe comprenant les cations métalliques,
- les groupes R représentent chacun indépendamment les uns des autres un radical choisi parmi les radicaux de formule générale V ou V' : 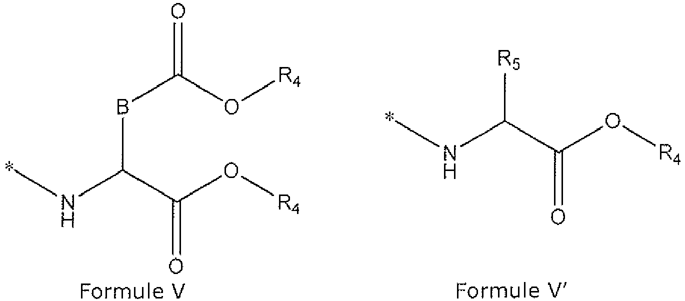 dans lesquelles, * indique le site de rattachement au co-polyaminoacide, et,
   - R₄ représente un radical en C8 à C30 linéaire ou ramifié, saturé ou insaturé, pouvant comporter des hétéroatomes ou un radical en C8 à C30 pouvant former des cycles carbonés ou pouvant comporter des hétéroatomes saturés, insaturés et/ou aromatiques, lesdits cycles pouvant être ortho-condensés ou péri-condensés,
   - B représente, indépendamment un groupe -CH₂- (unité aspartique) ou un groupe -CH₂-CH₂- (unité glutamique),
   - R₅ représente indépendamment un H, un alkyle linéaire ou ramifié en C1 à C4 ou un groupement benzyle, ou
- R est un radical en C8 à C30 linéaire ou ramifié, saturé ou insaturé, pouvant comporter des hétéroatomes ou un radical en C8 à C30 pouvant former des cycles carbonés ou pouvant comporter des hétéroatomes saturés, insaturés et/ou aromatiques, lesdits cycles pouvant être ortho-condensés ou péri-condensés,
- n/(n+m) est défini comme le taux de greffage molaire en radical hydrophobe des unités monomériques et est compris entre 1 et 50 % molaire,
- n + m représente le degré de polymérisation du co-polyaminoacide, c'est-à-dire le nombre moyen d'unités monomériques par chaîne de co-polyaminoacide et 5 ≤ n + m ≤ 1000.

Les radicaux R'₃, R₃, R"₃, R, R₄, R'₄, R"₄, R"'₄, R', R", R₅ et les groupes A et B, étant chacun indépendamment identiques ou différents d'une unité monomérique à l'autre.

Dans un mode de réalisation, le co-polyaminoacide porteur de charges carboxylates et substitué par des groupements hydrophobes est choisi parmi les co-polyaminoacides de formule IV : 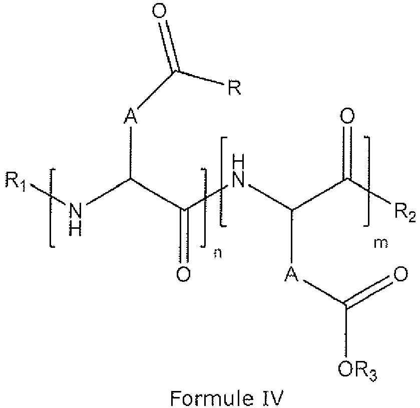 dans laquelle,
- A représente, indépendamment un groupe -CH₂- (unité aspartique) ou un groupe -CH₂-CH₂- (unité glutamique),
- R₁ est choisi dans le groupe constitué par un H, un groupe acyle linéaire en C2 à C10, un groupe acyle ramifié en C3 à C10, un benzyle, une unité « acide aminé » terminale et un pyroglutamate,
- R₂ représente un radical -NHR₁₁ dans lequel R₁₁ est choisi dans le groupe constitué par un H, un alkyle linéaire en C2 à C10 ou ramifié en C3 à C10, ou un benzyle ou un acyle en C2 à C30 linéaire ou ramifié et pouvant comporter des hétéroatomes choisis dans le groupe constitué par N, O et S,
- les groupes R₃ identiques ou différents sont choisis dans le groupe constitué par un H ou une entité cationique choisie dans le groupe comprenant les cations métalliques,
- les groupes R représentent chacun indépendamment les uns des autres un radical choisi parmi les radicaux de formule générale IV ou IV' : 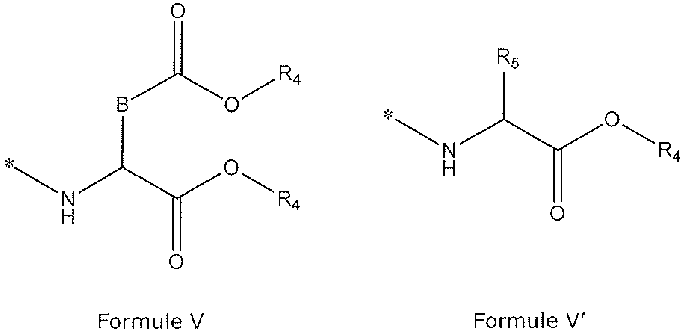 dans lesquelles, * indique le site de rattachement au co-polyaminoacide, et,
   - R₄ représente un radical en C8 à C30 linéaire ou ramifié, saturé ou insaturé, pouvant comporter des hétéroatomes ou un radical en C8 à C30 pouvant former des cycles carbonés ou pouvant comporter des hétéroatomes saturés, insaturés et/ou aromatiques, lesdits cycles pouvant être ortho-condensés ou péri-condensés,
   - B représente, indépendamment un groupe -CH₂- (unité aspartique) ou un groupe -CH₂-CH₂- (unité glutamique),
   - R₅ représente indépendamment un H, un alkyle linéaire ou ramifié en C1 à C4 ou un groupement benzyle, ou
- R est un radical en C8 à C30 linéaire ou ramifié, saturé ou insaturé, pouvant comporter des hétéroatomes ou un radical en C8 à C30 pouvant former des cycles carbonés ou pouvant comporter des hétéroatomes saturés, insaturés et/ou aromatiques, lesdits cycles pouvant être ortho-condensés ou péri-condensés,
- n/(n+m) est défini comme le taux de greffage molaire en radical hydrophobe des unités monomériques et est compris entre 1 et 50 % molaire,
- n + m représente le degré de polymérisation du co-polyaminoacide, c'est-à-dire le nombre moyen d'unités monomériques par chaîne de co-polyaminoacide et 5 ≤ n + m ≤ 1000.

Dans un mode de réalisation la composition selon l'invention est caractérisée en ce que le co-polyaminoacide est choisi parmi les co-polyaminoacides de formule I ou IV, dans lesquelles le groupe A est un groupe -CH₂- (unité aspartique).

Lorsque le co-polyaminoacide est constitué d'unités aspartiques, celles-ci peuvent subir des réarrangements structuraux.

Dans un mode de réalisation la composition selon l'invention est caractérisée en ce que les co-polyaminoacides de formule I ou IV peuvent en outre comprendre des unités monomériques de formule VI" et/ou VI' :

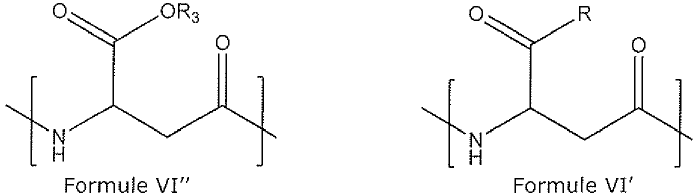

Dans un mode de réalisation, la composition selon l'invention est, caractérisée en ce que le co-polyaminoacide est choisi parmi les co-polyaminoacides de formules I ou IV, dans lesquelles le groupe A est un groupe -CH₂-CH₂- (unité glutamique).

Dans un mode réalisation, la composition selon l'invention est caractérisée en ce que le co-polyaminoacide est choisi parmi les co-polyaminoacides de formule I ou IV, dans laquelle R est choisi dans le groupe des radicaux issus des alcools hydrophobes.

Le radical R issu d'un alcool hydrophobe est obtenu par réaction d'une fonction alcool de l'alcool hydrophobe et une fonction acide carboxylique du co-polyaminoacide

Dans un mode de réalisation, le radical issu des alcools hydrophobes est choisi parmi les radicaux issus des alcools constitués d'une chaîne alkyle insaturée ou saturée comprenant de 8 à 18 carbones.

Dans un mode de réalisation, le radical issu des alcools hydrophobes est choisi dans le groupe constitué par les radicaux issus des alcools myristylique, cétylique, stéarylique, ou oléylique.

Dans un mode de réalisation, le radical issu des alcools hydrophobes est un radical issu des alcools dérivés du cholestérol.

Dans un mode de réalisation, le radical issu des alcools hydrophobes est choisi parmi les radicaux issus des alcools tocophérols.

Dans un mode de réalisation, le radical issu des alcools hydrophobes est choisi parmi les radicaux issus des alcools porteurs de groupe aryle.

Dans un mode de réalisation, le radical issu des alcools hydrophobes est choisi parmi les radicaux issus de l'alcool benzylique ou l'alcool phenéthylique.

Dans un mode de réalisation, le radical issu des alcools hydrophobes est choisi parmi les co-polyaminoacides de formule I, dans laquelle R est un radical issu de l'alcool laurylique.

Dans un mode de réalisation, le radical issu des alcools hydrophobes est un radical issu du tocophérol.

Dans un mode de réalisation, le radical issu des alcools hydrophobes est un radical issu du cholestérol.

Dans un mode de réalisation la composition selon l'invention est caractérisée en ce que le co-polyaminoacide est choisi parmi les co-polyaminoacides de formule I ou IV, dans laquelle le groupe R est un radical de type -L-R"', le radical R"' étant issu d'un acide hydrophobe et le radical L étant un bras de liaison comprenant de 2 à 10 carbones linéaire ou ramifié et pouvant comporter des hétéroatomes choisis dans le groupe constitué par N, O et S issu d'un diol, d'une diamine ou d'un amino-alcool.

Le radical issu d'un acide hydrophobe étant issu de la réaction de la fonction acide de l'acide hydrophobe et d'une fonction alcool ou amine de la diamine, du diol ou de l'amino-alcool.

Le radical L étant issu d'une part de de la réaction de la fonction acide de l'acide hydrophobe et d'une fonction alcool ou amine de la diamine, du diol ou de l'amino-alcool et d'autre part de la réaction entre une fonction acide carboxylique du co-polyaminoacide et d'une fonction alcool ou amine de la diamine, du diol ou de l'amino-alcool.

Dans un mode de réalisation les acides hydrophobes sont choisis dans le groupe constitué par les acides gras.

Dans un mode de réalisation, les acides gras sont choisis dans le groupe constitué par les acides gras linéaires.

Dans un mode de réalisation, les acides gras linéaires sont choisis dans le groupe constitué par l'acide caproïque, l'acide oenanthique, l'acide caprylique, l'acide caprique, l'acide nonanoïque, l'acide décanoïque, l'acide undécanoïque, l'acide dodécanoïque, l'acide palmitique, l'acide stéarique, l'acide arachidique, l'acide béhénique, l'acide tricosanoïque, l'acide lignocérique, l'acide heptacosanoïque et l'acide octacosanoïque.

Dans un mode de réalisation, les acides gras sont choisis dans le groupe constitué par les acides gras insaturés.

Dans un mode de réalisation, les acides gras insaturés sont choisis dans le groupe constitué par l'acide myristoléique, l'acide palmitoléique, l'acide oléique, l'acide élaidique, l'acide linoléique, l'acide alpha-linoléique, l'acide arachidonique, l'acide eicosapentaenoïque, l'acide erucique et l'acide docosahexaenoïque.

Dans un mode de réalisation, les acides gras sont choisis dans le groupe constitué par les acides de la bile et leurs dérivés.

Dans un mode de réalisation, les acides de la bile et leurs dérivés sont choisis dans le groupe constitué par l'acide cholique, l'acide déhydrocholique, l'acide désoxycholique et l'acide chénodésoxycholique.

Dans un mode de réalisation les diamines sont choisies dans le groupe constitué par l'éthylène diamine et la lysine et ses dérivés.

Dans un mode de réalisation, les diamines sont choisies dans le groupe constitué par la diéthylèneglycoldiamine et la triéthylèneglycoldiamine.

Dans un mode de réalisation les diols sont choisis dans le groupe constitué par glycérol, le diglycérol et le triglycérol.

Dans un mode de réalisation, les dialcools sont choisis dans le groupe constitué par le diéthylèneglycol et le triéthylèneglycol.

Dans un mode de réalisation les amino-alcools sont choisis dans le groupe constitué par l'éthanolamine, l'amino-2-propanol, l'isopropanolamine, le 3-amino-1,2-propanediol, la diéthanolamine, la diisopropanolamine, la trométhamine (Tris) et le 2-(2-aminoéthoxy)éthanol.

Dans un mode de réalisation, les alcoolamines sont choisies dans le groupe constitué par les acides aminés réduits.

Dans un mode de réalisation les acides aminés réduits sont choisis dans le groupe constitué par l'alaninol, le valinol, le leucinol, l'isoleucinol, le prolinol, le phénylalaninol, sérinol et thréoninol.

Dans un mode de réalisation, les alcoolamines sont choisies dans le groupe constitué par les acides aminés chargés.

Dans un mode de réalisation, les acides aminés chargés sont choisis dans le groupe constitué par la sérine et la thréonine.

Dans un mode de réalisation, L est un radical trivalent.

Dans un mode de réalisation, L est un radical trivalent choisi dans le groupe constitué par les triamines, les dialcool-amines, les diamine-alcools, les diamine-acides.

Dans un mode de réalisation, la composition selon l'invention est caractérisée en ce que le co-polyaminoacide est choisi parmi les co-polyaminoacides de formule I ou IV, dans laquelle R₂ est le -N-morpholyl.

Dans un mode de réalisation, la composition selon l'invention est caractérisée en ce que le co-polyaminoacide est choisi parmi les co-polyaminoacides de formule I, dans laquelle le groupement R₂ est un radical issu d'un acide aminé et est choisi parmi les radicaux de formule VII : 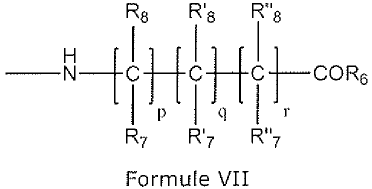 dans laquelle,
- R₆ est -OH, -OR₉ ou -NHR₁₀, et
- R₇, R'₇, R"₇, R₈, R'₈, R"₈, R₉ et R₁₀ identiques ou différents représentent indépendamment un H, un alkyle linéaire en C2 à C10, un alkyle ramifié en C3 à C10 ou un benzyle, avec 0 ≤ p ≤ 3, 0 ≤ q ≤ 3, 0 ≤ r ≤ 3 et 1 ≤ p+q+r ≤ 10.

Dans un mode réalisation, la composition selon l'invention est caractérisée en ce que le co-polyaminoacide est choisi parmi les co-polyaminoacides de formules I ou IV, dans lesquelles R_{4,} R'₄ et/ou R"₄, identiques ou différents, sont choisis dans le groupe des radicaux issus des alcools hydrophobes.

Les radicaux issus d'alcools hydrophobes sont tels que définis ci-dessus, et sont obtenus par réaction d'une fonction acide portée par un précurseur d'un radical de formule III, III', V, ou V' et la fonction alcool de l'alcool hydrophobe.

Dans un mode de réalisation, le radical issu des alcools hydrophobes est choisi parmi les radicaux issus des alcools constitués d'une chaîne alkyle insaturée ou saturée comprenant de 8 à 18 carbones.

Dans un mode de réalisation, le radical issu des alcools hydrophobes est choisi dans le groupe constitué par les radicaux issus des alcools myristylique, cétylique, stéarylique, oléylique.

Dans un mode de réalisation, le radical issu des alcools hydrophobes est un radical issu des dérivés du cholestérol.

Dans un mode de réalisation, le radical issu des alcools hydrophobes est choisi parmi les radicaux issus des tocophérols,

Dans un mode de réalisation, le radical issu des alcools hydrophobes est choisi parmi les radicaux issus des alcools porteurs de groupe aryle.

Dans un mode de réalisation, le radical issu des alcools hydrophobes est choisi parmi les radicaux issus de l'alcool benzylique ou l'alcool phenéthylique.

Dans un mode de réalisation, le radical issu des alcools hydrophobes est choisi parmi les co-polyaminoacides de formule I ou IV, dans laquelle R est un radical issu de l'alcool laurylique.

Dans un mode de réalisation, le radical issu des alcools hydrophobes est un radical issu du cholestérol.

Dans un mode de réalisation, le radical issu des alcools hydrophobes est un radical issu du tocophérol.

Dans un mode de réalisation, la composition selon l'invention est caractérisée en ce que le co-polyaminoacide est choisi parmi les co-polyaminoacides de formules I ou IV, dans lesquelles le groupe R₅ est un radical isobutyl.

Dans un mode de réalisation, la composition selon l'invention est caractérisée en ce que le co-polyaminoacide est choisi parmi les co-polyaminoacides de formules I ou IV, dans lesquelles le groupe R₅ est un H.

Dans un mode de réalisation, la composition selon l'invention est caractérisée en ce que le co-polyaminoacide est choisi parmi les co-polyaminoacides de formules I ou IV, dans lesquelles le groupe R₅ est choisi dans le groupe constitué par les radicaux méthyl, isopropyl, *sec*-butyl et benzyle.

Dans un mode de réalisation, la composition selon l'invention est caractérisée en ce que le co-polyaminoacide est choisi parmi les co-polyaminoacides de formules I ou IV, dans lesquelles n + m est compris entre 5 et 500.

Dans un mode de réalisation, la composition selon l'invention est caractérisée en ce que le co-polyaminoacide est choisi parmi les co-polyaminoacides de formules I ou IV, dans lesquelles n + m est compris entre 5 et 250.

Dans un mode de réalisation, la composition selon l'invention est caractérisée en ce que le co-polyaminoacide est choisi parmi les co-polyaminoacides de formules I ou IV, dans lesquelles n + m est compris entre 5 et 100.

Dans un mode de réalisation, la composition selon l'invention est caractérisée en ce que le co-polyaminoacide est choisi parmi les co-polyaminoacides de formules I ou IV, dans lesquelles n + m est compris entre 5 et 50.

Dans un mode de réalisation, la composition selon l'invention est caractérisée en ce que le co-polyaminoacide est choisi parmi les co-polyaminoacides de formules I ou IV, dans lesquelles n + m est compris entre 5 et 25.

Dans un mode de réalisation, la composition selon l'invention est caractérisée en ce que le co-polyaminoacide est choisi parmi les co-polyaminoacides de formules I ou IV, dans lesquelles n + m est compris entre 10 et 500.

Dans un mode de réalisation, la composition selon l'invention est caractérisée en ce que le co-polyaminoacide est choisi parmi les co-polyaminoacides de formules 1 ou IV, dans lesquelles n + m est compris entre 15 et 250.

Dans un mode de réalisation, la composition selon l'invention est caractérisée en ce que le co-polyaminoacide est choisi parmi les co-polyaminoacides de formules I ou IV, dans lesquelles n/(n+m) est compris entre 1 et 30 % molaire.

Dans un mode de réalisation, la composition selon l'invention est, caractérisée en ce que le co-polyaminoacide est choisi parmi les co-polyaminoacides de formules I ou IV, dans lesquelles n/(n+m) est compris entre 1 et 20 % molaire.

Dans un mode de réalisation, la composition selon l'invention est, caractérisée en ce que le co-polyaminoacide est choisi parmi les co-polyaminoacides de formules I ou IV, dans lesquelles n/(n+m) est compris entre 1 et 10 % molaire.

Dans un mode de réalisation, la composition selon l'invention est, caractérisée en ce que le co-polyaminoacide est choisi parmi les co-polyaminoacides de formules I ou IV, dans lesquelles n/(n+m) est compris entre 1 et 5 % molaire.

Dans un mode de réalisation, la composition selon l'invention est caractérisée en ce que le co-polyaminoacide est issu d'un polyaminoacide obtenu par polymérisation par ouverture de cycle d'un dérivé de N-carboxyanhydride d'acide glutamique ou d'un dérivé de N-carboxyanhydride d'acide aspartique,

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique comme décrit dans l'article de revue Adv. Polym. Sci. 2006, 202, 1-18 (Deming, T.J.).

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique en utilisant comme initiateur un complexe organométallique d'un métal de transition comme décrit dans la publication Nature 1997, 390, 386-389 (Deming, T.J.).

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique en utilisant comme initiateur l'ammoniaque ou une amine primaire comme décrit dans le brevet FR 2,801,226 (Touraud, F. ; et al.).

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique en utilisant comme initiateur l'hexaméthyldisilazane comme décrit dands la publication J. Am. Chem. Soc. 2007, 129, 14114-14115 (Lu H. ; et al.) ou une amine silylée comme décrit dans la publication J. Am. Chem. Soc. 2008, 130, 12562-12563 (Lu H. ; et al.).

Dans un mode de réalisation, la composition selon l'invention est caractérisée en ce que le co-polyaminoacide est obtenu par synthèse peptidique sur support en utilisant un synthétiseur à peptides.

Dans un mode de réalisation, la composition selon l'invention est caractérisée en ce que le co-polyaminoacide est obtenu par synthèse peptidique en phase liquide.

Dans un mode de réalisation, la composition selon l'invention est caractérisée en ce que le co-polyaminoacide est obtenu par greffage d'un groupe hydrophobe sur un poly-L-glutamique acide ou poly-L-aspartique acide en utilisant les procédés de formation de liaison amide bien connus de l'homme de l'art.

Dans un mode de réalisation, la composition selon l'invention est caractérisée en ce que le co-polyaminoacide est obtenu par greffage d'un groupe hydrophobe sur un poly-L-glutamique acide ou poly-L-aspartique acide comme décrit dans le brevet FR 2,840,614 (Ping, C.U. ; et al.).

On entend par insuline basale dont le point isoélectrique est compris entre 5,8 et 8,5 une insuline insoluble à pH 7 et dont la durée d'action est comprise entre 8 et 24 heures ou supérieure dans les modèles standards de diabète.

Ces insulines basales dont le point isoélectrique est compris entre 5,8 et 8,5 sont des insulines recombinantes dont la structure primaire a été modifiée principalement par introduction d'aminoacides basiques comme l'Arginine ou la Lysine. Elles sont décrites par exemple dans les brevets, demandes de brevets ou publications suivants WO 2003/053339, WO 2004/096854, US 5,656,722 et US 6,100,376.

Dans un mode de réalisation, l'insuline basale dont le point isoélectrique est compris entre 5,8 et 8,5 est l'insuline glargine.

Dans un mode de réalisation les compositions selon l'invention comprennent entre 40 et 500 UI/mL d'insuline basale dont le point isoélectrique est compris entre 5,8 et 8,5.

Dans un mode de réalisation les compositions selon l'invention comprennent 40 UI/mL d'insuline basale dont le point isoélectrique est compris entre 5,8 et 8,5.

Dans un mode de réalisation, les compositions selon l'invention comprennent 100 UI/mL (soit environ 3,6 mg/mL) d'insuline basale dont le point isoélectrique est compris entre 5,8 et 8,5.

Dans un mode de réalisation, les compositions selon l'invention comprennent 200 UI/mL d'insuline basale dont le point isoélectrique est compris entre 5,8 et 8,5.

Dans un mode de réalisation, les compositions selon l'invention comprennent 300 UI/mL d'insuline basale dont le point isoélectrique est compris entre 5,8 et 8,5.

Dans un mode de réalisation, les compositions selon l'invention comprennent 400 UI/mL d'insuline basale dont le point isoélectrique est compris entre 5,8 et 8,5.

Dans un mode de réalisation, les compositions selon l'invention comprennent 500 UI/mL d'insuline basale dont le point isoélectrique est compris entre 5,8 et 8,5.

Dans un mode de réalisation, le ratio massique entre l'insuline basale, dont le point isoélectrique est compris entre 5,8 et 8,5, et le co-polyaminoacide substitué, soit co-polyaminoacide substitué/insuline basale, est compris entre 0,2 et 30.

Dans un mode de réalisation, le ratio massique est compris entre 0,2 et 15.

Dans un mode de réalisation, le ratio massique est compris entre 0,2 et 10.

Dans un mode de réalisation, le ratio massique est compris entre 0,2 et 4.

Dans un mode de réalisation, le ratio massique est compris entre 0,2 et 3.

Dans un mode de réalisation, le ratio massique est compris entre 0,2 et 2.

Dans un mode de réalisation, le ratio massique est compris entre 0,2 et 1.

Dans un mode de réalisation, le ratio massique est égal à 1.

Dans un mode de réalisation, la concentration en co-polyaminoacide substitué est au plus de 100 mg/mL.

Dans un mode de réalisation, la concentration en co-polyaminoacide substitué est au plus de 80 mg/mL.

Dans un mode de réalisation, la concentration en co-polyaminoacide substitué est au plus de 60 mg/mL.

Dans un mode de réalisation, la concentration en co-polyaminoacide substitué est au plus de 40 mg/mL.

Dans un mode de réalisation, la concentration en co-polyaminoacide substitué est au plus de 20 mg/mL.

Dans un mode de réalisation, la concentration en co-polyaminoacide substitué est au plus de 10 mg/mL.

Dans un mode de réalisation, les compositions selon l'invention comprennent en outre une insuline prandiale. Les insulines prandiales sont solubles à pH 7.

On entend par insuline prandiale une insuline dite rapide ou « regular ».

Les insulines prandiales dites rapides sont des insulines qui doivent répondre aux besoins provoqués par l'ingestion des protéines et des glucides durant un repas, elles doivent agir en moins de 30 minutes.

Dans un mode de réalisation, l'insuline prandiale dite « regular » est de l'insuline humaine.

Dans un mode de réalisation, l'insuline est une insuline humaine recombinante telle que décrite dans la Pharmacopée Européenne et la Pharmacopée américaine.

L'insuline humaine est par exemple commercialisée sous les marques Humulin® (ELI LILLY) et Novolin® (NOVO NORDISK).

Les insulines prandiales dites très rapides (fast acting) sont des insulines qui sont obtenues par recombinaison et dont la structure primaire a été modifiée pour diminuer leur temps d'action.

Dans un mode de réalisation, les insulines prandiales dites rapides (fast acting) sont choisies dans le groupe comprenant l'insuline lispro (Humalog®), l'insuline glulisine (Apidra®) et l'insuline aspart (NovoLog®).

Dans un mode de réalisation, l'insuline prandiale est l'insuline lispro.

Dans un mode de réalisation, l'insuline prandiale est l'insuline glulisine.

Dans un mode de réalisation, l'insuline prandiale est l'insuline aspart.

Dans un mode de réalisation les compositions selon l'invention comprennent au total entre 40 et 800 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total entre 40 et 500 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total 800 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total 700 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total 600 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total 500 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total 400 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total 300 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total 200 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total 100 UI/mL d'insuline avec une combinaison d'insuline prandiale et d'insuline basale dont le point isoélectrique est compris entre 5,8 et 8,5.

Dans un mode de réalisation, les compositions selon l'invention comprennent au total 40 UI/mL d'insuline avec une combinaison d'insuline prandiale et d'insuline basale dont le point isoélectrique est compris entre 5,8 et 8,5.

Les proportions entre l'insuline basale dont le point isoélectrique est compris entre 5,8 et 8,5 et l'insuline prandiale sont par exemple en pourcentage de 25/75, 30/70, 40/60, 50/50, 60/40, 70/30, 80/20, 90/10 pour des formulations telles que décrites ci-dessus comprenant de 40 à 800 UI/mL. Cependant toute autre proportion peut être réalisée.

Dans un mode de réalisation, les compositions selon l'invention comprennent en outre une hormone gastrointestinale.

On entend par « hormones gastrointestinales », les hormones choisies dans le groupe constitué par le GLP-1 (Glucagon like peptide-1) et le GIP (Glucose-dependent insulinotropic peptide), l'oxyntomoduline (un dérivé du proglucagon), le peptide YY, l'amyline, la cholecystokinine, le polypeptide pancréatique (PP), la ghreline et l'entérostatine, leurs analogues ou dérivés et/ou leurs sels pharmaceutiquement acceptables.

Dans un mode de réalisation, les hormones gastro-intestinales sont des analogues ou dérivés de GLP-1 choisis dans le groupe constitué par l'exenatide ou Byetta®, développé par ELI LILLY & CO et AMYLIN PHARMACEUTICALS, le liraglutide ou Victoza® développé par NOVO NORDISK, ou le lixisenatide ou Lyxumia® developpé par SANOFI-AVENTIS, leurs analogues ou dérivés et leurs sels pharmaceutiquement acceptables.

Dans un mode de réalisation, l'hormone gastrointestinale est l'exenatide ou Byetta® ses analogues ou dérivés et leurs sels pharmaceutiquement acceptables.

Dans un mode de réalisation, l'hormone gastrointestinale est le liraglutide ou Victoza® ses analogues ou dérivés et leurs sels pharmaceutiquement acceptables.

Dans un mode de réalisation, l'hormone gastrointestinale est le lixisenatide ou Lyxumia® ses analogues ou dérivés et leurs sels pharmaceutiquement acceptables.

On entend par « analogue », lorsqu'il est utilisé par référence à un peptide ou une protéine, un peptide ou une protéine, dans lequel un ou plusieurs résidus d'acides aminés constitutifs ont été substitués par d'autres résidus d'acides aminés et/ou dans lequel un ou plusieurs résidus d'acides aminés constitutifs ont été supprimés et/ou dans lequel un ou plusieurs résidus d'acides aminés constitutifs ont été ajoutés. Le pourcentage d'homologie admis pour la présente définition d'un analogue est de 50 %.

On entend par « dérivé », lorsqu'il est utilisé par référence à un peptide ou une protéine, un peptide ou une protéine ou un analogue chimiquement modifié par un substituant qui n'est pas présent dans le peptide ou la protéine ou l'analogue de référence, c'est-à-dire un peptide ou une protéine qui a été modifié par création de liaisons covalentes, pour introduire des substituants.

Dans un mode de réalisation, le substituant est choisi dans le groupe constitué des chaines grasses.

Dans un mode de réalisation, la concentration en hormone gastrointestinale est comprise dans un intervalle de 0,01 à 10 mg/mL.

Dans un mode de réalisation, la concentration en exenatide, ses analogues ou dérivés et leurs sels pharmaceutiquement acceptables est comprise dans un intervalle de 0,05 à 0,5 mg/mL.

Dans un mode de réalisation, la concentration en liraglutide, ses analogues ou dérivés et leurs sels pharmaceutiquement acceptables est comprise dans un intervalle de 1 à 10 mg/mL.

Dans un mode de réalisation, la concentration en lixisenatide, ses analogues ou dérivés et leurs sels pharmaceutiquement acceptables est comprise dans un intervalle de 0,01 à 1 mg/mL.

Dans un mode de réalisation, les compositions selon l'invention sont réalisées par mélange de solutions commerciales d'insuline basale dont le point isoélectrique est compris entre 5,8 et 8,5 et de solutions commerciales de GLP-1, d'analogue ou de dérivé de GLP-1 en ratios volumiques compris dans un intervalle de 10/90 à 90/10.

Dans un mode de réalisation, la composition selon l'invention comprend une dose journalière d'insuline basale et une dose journalière d'hormone gastro-intestinale.

Dans un mode de réalisation, les compositions selon l'invention comprennent entre 500 UI/mL et 40 UI/mL d'insuline basale dont le point isoélectrique est compris entre 5,8 et 8,5 et, entre 0,05 et 0,5 mg/mL d'exenatide.

Dans un mode de réalisation, les compositions selon l'invention comprennent entre 500 UI/mL et 40 UI/mL d'insuline basale dont le point isoélectrique est compris entre 5,8 et 8,5 et, de 1 à 10 mg/mL de liraglutide.

Dans un mode de réalisation, les compositions selon l'invention comprennent entre 500 UI/mL et 40 UI/mL d'insuline basale dont le point isoélectrique est compris entre 5,8 et 8,5 et, de 0,01 à 1 mg/mL de lixisenatide.

Dans un mode de réalisation, les compositions selon l'invention comprennent 500 UI/mL d'insuline basale dont le point isoélectrique est compris entre 5,8 et 8,5 et, de 0,05 à 0,5 mg/mL d'exenatide.

Dans un mode de réalisation, les compositions selon l'invention comprennent 500 UI/mL d'insuline basale dont le point isoélectrique est compris entre 5,8 et 8,5 et, de 1 à 10 mg/mL de liraglutide.

Dans un mode de réalisation, les compositions selon l'invention comprennent 500 UI/mL d'insuline basale dont le point isoélectrique est compris entre 5,8 et 8,5 et, de 0,01 à 1 mg/mL de lixisenatide.

Dans un mode de réalisation, les compositions selon l'invention comprennent 400 UI/mL d'insuline basale dont le point isoélectrique est compris entre 5,8 et 8,5 et, de 0,05 à 0,5 mg/mL d'exenatide.

Dans un mode de réalisation, les compositions selon l'invention comprennent 400 UI/mL d'insuline basale dont le point isoélectrique est compris entre 5,8 et 8,5 et, de 1 à 10 mg/mL de liraglutide.

Dans un mode de réalisation, les compositions selon l'invention comprennent 400 UI/mL d'insuline basale dont le point isoélectrique est compris entre 5,8 et 8,5 et, de 0,01 à 1 mg/mL de lixisenatide.

Dans un mode de réalisation, les compositions selon l'invention comprennent 300 UI/mL d'insuline basale dont le point isoélectrique est compris entre 5,8 et 8,5 et, de 0,05 à 0,5 mg/mL d'exenatide.

Dans un mode de réalisation, les compositions selon l'invention comprennent 300 UI/mL d'insuline basale dont le point isoélectrique est compris entre 5,8 et 8,5 et, de 1 à 10 mg/mL de liraglutide,

Dans un mode de réalisation, les compositions selon l'invention comprennent 300 UI/mL d'insuline basale dont le point isoélectrique est compris entre 5,8 et 8,5 et, de 0,01 à 1 mg/mL de lixisenatide.

Dans un mode de réalisation, les compositions selon l'invention comprennent 200 UI/mL d'insuline basale dont le point isoélectrique est compris entre 5,8 et 8,5 et, de 0,05 à 0,5 mg/mL d'exenatide.

Dans un mode de réalisation, les compositions selon l'invention comprennent 200 UI/mL d'insuline basale dont le point isoélectrique est compris entre 5,8 et 8,5 et, de 1 à 10 mg/mL de liraglutide.

Dans un mode de réalisation, les compositions selon l'invention comprennent 200 UI/mL d'insuline basale dont le point isoélectrique est compris entre 5,8 et 8,5 et, de 0,01 à 1 mg/mL de lixisenatide.

Dans un mode de réalisation, les compositions selon l'invention comprennent 100 UI/mL (soit environ 3,6 mg/mL) d'insuline basale dont le point isoéfectrique est compris entre 5,8 et 8,5 et, de 0,05 à 0,5 mg/mL d'exenatide.

Dans un mode de réalisation, les compositions selon l'invention comprennent 100 UI/mL (soit environ 3,6 mg/mL) d'insuline basale dont le point isoélectrique est compris entre 5,8 et 8,5 et, de 1 à 10 mg/mL de liraglutide.

Dans un mode de réalisation, les compositions selon l'invention comprennent 100 UI/mL (soit environ 3,6 mg/mL) d'insuline basale dont le point isoélectrique est compris entre 5,8 et 8,5 et, de 0,01 à 1 mg/mL de lixisenatide,

Dans un mode de réalisation, les compositions selon l'invention comprennent 40 UI/mL d'insuline basale dont le point isoélectrique est compris entre 5,8 et 8,5 et, de 0,05 à 0,5 mg/mL d'exenatide.

Dans un mode de réalisation, les compositions selon l'invention comprennent 40 UI/mL d'insuline basale dont le point isoélectrique est compris entre 5,8 et 8,5 et, de 1 à 10 mg/mL de liraglutide.

Dans un mode de réalisation, les compositions selon l'invention comprennent 40 UI/mL d'insuline basale dont le point isoélectrique est compris entre 5,8 et 8,5 et, de 0,01 à 1 mg/mL de lixisenatide.

Dans un mode de réalisation, les compositions selon l'invention comprennent en outre des sels de zinc à une concentration comprise entre 0 et 5000 µM.

Dans un mode de réalisation, les compositions selon l'invention comprennent en outre des sels de zinc à une concentration comprise entre 0 et 4000 µM.

Dans un mode de réalisation, les compositions selon l'invention comprennent en outre des sels de zinc à une concentration comprise entre 0 et 3000 µM.

Dans un mode de réalisation, les compositions selon l'invention comprennent en outre des sels de zinc à une concentration comprise entre 0 et 2000 µM.

Dans un mode de réalisation, les compositions selon l'invention comprennent en outre des sels de zinc à une concentration comprise entre 0 et 1000 µM.

Dans un mode de réalisation, les compositions selon l'invention comprennent en outre des sels de zinc à une concentration comprise entre 50 et 600 µM.

Dans un mode de réalisation, les compositions selon l'invention comprennent en outre des sels de zinc à une concentration comprise entre 100 et 500 µM.

Dans un mode de réalisation, les compositions selon l'invention comprennent en outre des sels de zinc à une concentration comprise entre 200 et 500 µM.

Dans un mode de réalisation, les compositions selon l'invention comprennent en outre des tampons.

Dans un mode de réalisation, les compositions selon l'invention comprennent des tampons à des concentrations comprises entre 0 et 100 mM.

Dans un mode de réalisation, les compositions selon l'invention comprennent des tampons à des concentrations comprises entre 15 et 50 mM.

Dans un mode de réalisation, les compositions selon l'invention comprennent un tampon choisi dans le groupe constitué par un tampon phosphate, le Tris (trishydroxyméthylaminométhane) ou le citrate de sodium.

Dans un mode de réalisation, le tampon est le phosphate de sodium.

Dans un mode de réalisation, le tampon est le Tris (trishydroxyméthylaminométhane).

Dans un mode de réalisation, le tampon est le citrate de sodium.

Dans un mode de réalisation, les compositions selon l'invention comprennent en outre des conservateurs.

Dans un mode de réalisation, les conservateurs sont choisis dans le groupe constitué par le m-crésol et le phénol seuls ou en mélange.

Dans un mode de réalisation, la concentration des conservateurs est comprise entre 10 et 50 mM.

Dans un mode de réalisation, la concentration des conservateurs est comprise entre 10 et 40 mM.

Dans un mode de réalisation, les compositions selon l'invention comprennent en outre un tensioactif.

Dans un mode de réalisation, le tensioactif est choisi dans le groupe constitué par le propylène glycol ou le polysorbate.

Les compositions selon l'invention peuvent en outre comprendre des additifs tels que des agents de tonicité.

Dans un mode de réalisation, les agents de tonicité sont choisis dans le groupe constitué par la glycérine, le chlorure de sodium, le mannitol et la glycine.

Les compositions selon l'invention peuvent comprendre en outre tous les excipients conformes aux pharmacopées et compatibles avec les insulines utilisées aux concentrations d'usage.

La demande concerne également une formulation pharmaceutique selon l'invention, caractérisée en ce qu'elle est obtenue par séchage et/ou lyophilisation.

Dans le cas des libérations locale et systémique, les modes d'administration envisagés sont par voie intraveineuse, sous-cutanée, intradermique ou intramusculaire.

Les voies d'administration transdermique, orale, nasale, vaginale, oculaire, buccale, pulmonaire sont également envisagées.

L'invention concerne également des formulations unidoses à pH compris entre 7 et 7,8 comprenant une insuline basale dont le point isoélectrique est compris entre 5,8 et 8.5 et un co-polyaminoacide substitué tel que défini précédemment.

L'invention concerne également des formulations unidoses à pH compris entre 7 et 7,8 comprenant une insuline basale dont le point isoélectrique est compris entre 5,8 et 8,5, une insuline prandiale et un co-polyaminoacide substitué tel que décrit précédemment.

L'invention concerne également des formulations unidoses à pH compris entre 7 et 7,8 comprenant une insuline basale dont le point isoélectrique est compris entre 5,8 et 8,5, une hormone gastrointestinale et un co-polyaminoacide substitué tels que décrits précédemment.

L'invention concerne également des formulations unidoses à pH compris entre 7 et 7,8 comprenant une insuline basale dont le point isoélectrique est compris entre 5,8 et 8,5, une insuline prandiale, une hormone gastrointestinale, telle que définie précédemment et un co-polyaminoacide substitué tel que défini précédemment.

Dans un mode de réalisation, les formulations sont sous forme d'une solution injectable.

Dans un mode de réalisation, l'insuline basale dont le point isoélectrique est compris entre 5,8 et 8,5 est l'insuline glargine.

Dans un mode de réalisation, l'insuline prandiale est l'insuline humaine.

Dans un mode de réalisation, l'insuline est une insuline humaine recombinante telle que décrite dans la Pharmacopée Européenne et la Pharmacopée américaine.

Dans un mode de réalisation, l'insuline prandiale est choisie dans le groupe comprenant l'insuline lispro (Humalog®), l'insuline glulisine (Apidra®) et l'insuline aspart (NovoLog®).

Dans un mode de réalisation, l'insuline prandiale est l'insuline lispro.

Dans un mode de réalisation, l'insuline prandiale est l'insuline glulisine.

Dans un mode de réalisation, l'insuline prandiale est l'insuline aspart.

Dans un mode de réalisation, le GLP-1, analogue ou dérivé de GLP-1 est choisi dans le groupe comprenant exenatide (Byetta®), liraglutide (Victoza®), lixisenatide (Lyxumia®) ou l'un de leurs dérivés.

Dans un mode de réalisation, l'hormone gastrointestinale est l'exenatide,

Dans un mode de réalisation, l'hormone gastrointestinale est le liraglutide.

Dans un mode de réalisation, l'hormone gastrointestinale est le lixisenatide.

La solubilisation à pH compris entre 6,6 et 7,8 des insulines basales dont le point isoélectrique est compris entre 5,8 et 8,5, par les co-polyaminoacides substitués de formule I ou IV, peut être constatée et contrôlée de manière simple, à l'oeil nu, grâce à un changement d'aspect de la solution.

La solubilisation à pH compris entre 7 et 7,8 des insulines basales dont le point isoélectrique est compris entre 5,8 et 8,5, par les co-polyaminoacides substitués de formule I ou IV, peut être constatée et contrôlée de manière simple, à l'oeil nu, grâce à un changement d'aspect de la solution.

Par ailleurs et de façon toute aussi importante, la demanderesse a pu vérifier qu'une insuline basale dont le point isoélectrique est compris entre 5,8 et 8,5, solubilisée à pH compris entre 6,6 et 7,8 en présence d'un co-polyaminoacide substitué de formule I ou IV n'avait en rien perdu son action d'insuline lente que ce soit seule ou en combinaison avec une insuline prandiale ou une hormone gastrointestinale.

La demanderesse a également pu vérifier qu'une insuline prandiale mélangée à pH compris entre 6,6 et 7,8 en présence d'un co-polyaminoacide de formule I ou IV et d'une insuline basale dont le point isoélectrique est compris entre 5,8 et 8,5, n'avait en rien perdu son action d'insuline rapide.

La préparation d'une composition selon l'invention présente l'avantage de pouvoir être réalisée par simple mélange d'une solution aqueuse d'insuline basale dont le point isoélectrique est compris entre 5,8 et 8,5, et d'un co-polyaminoacide substitué de formule I ou IV, en solution aqueuse ou sous forme lyophilisée. Si nécessaire, le pH de la préparation est ajusté à pH 7.

La préparation d'une composition selon l'invention présente l'avantage de pouvoir être réalisée par simple mélange d'une solution aqueuse d'insuline basale dont le point isoélectrique est compris entre 5,8 et 8,5, d'une solution d'insuline prandiale, et d'un co-polyaminoacide substitué de formule I ou IV, en solution aqueuse ou sous forme lyophilisée. Si nécessaire, le pH de la préparation est ajusté à pH 7.

La préparation d'une composition selon l'invention présente l'avantage de pouvoir être réalisée par simple mélange d'une solution aqueuse d'insuline basale dont le point isoélectrique est compris entre 5,8 et 8,5, d'une solution de GLP-1, un analogue ou un dérivé de GLP-1, et d'un co-polyaminoacide substitué de formule I ou IV, en solution aqueuse ou sous forme lyophilisée, Si nécessaire, le pH de la préparation est ajusté à pH 7.

La préparation d'une composition selon l'invention présente l'avantage de pouvoir être réalisée par simple mélange d'une solution aqueuse d'insuline basale dont le point isoélectrique est compris entre 5,8 et 8,5, d'une solution d'insuline prandiale, d'une solution de GLP-1 ou d'un analogue ou dérivé de GLP-1 et d'un co-polyaminoacide substitué de formule I ou IV, en solution aqueuse ou sous forme lyophilisée. Si nécessaire, le pH de la préparation est ajusté à pH 7.

Dans un mode de réalisation le mélange d'insuline basale et de co-polyaminoacide substitué est concentré par ultrafiltration avant le mélange avec l'insuline prandiale en solution aqueuse ou sous forme lyophilisée.

Si nécessaire, la composition du mélange est ajustée en excipients tels que glycérine, m-crésol, chlorure de zinc, et polysorbate (Tween®) par ajout de solutions concentrées de ces excipients au sein du mélange. Si nécessaire, le pH de la préparation est ajusté à 7.

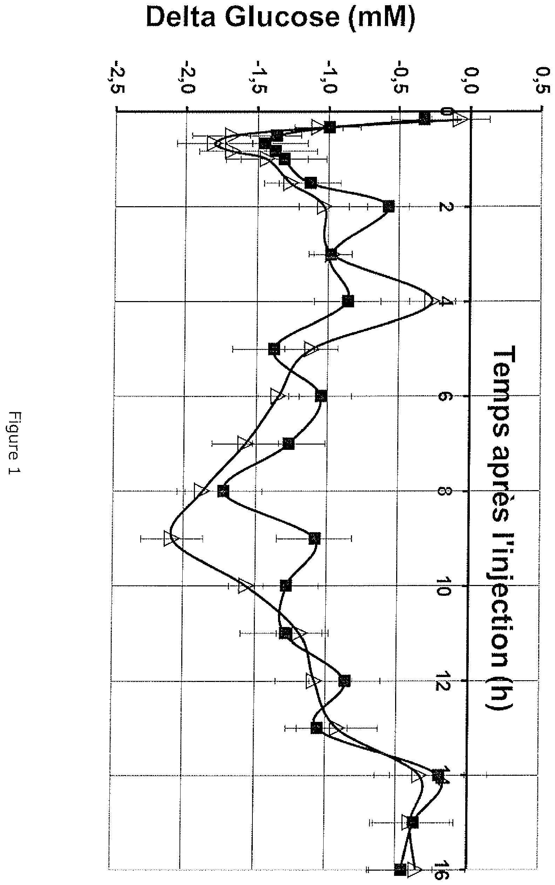

La figure 1 illustre les résultats de l'étude pharmacodynamique obtenus avec une composition selon l'invention en comparaison avec une administration séquentielle des mêmes insulines.

Sont représentées sur la Figure 1, les courbes moyennes et écart-type de la moyenne pour les administrations séquentielles de Humalog®(100 UI/mL) et Lantus®(100 UI/mL) (carrés pleins) en comparaison avec l'administration d'une composition selon l'invention co-polyaminoacide 5/insuline glargine (300 UI/mL)/insuline lispro (100 UI/mL) (triangles).

### Exemples

### Partie A Synthèse des co-polyaminoacides

**Tableau 1 : liste des co-polyaminoacides synthétisés**

| N° co-polyaminoacide | Formule | Degré de polymérisation (n+m) | Taux de greffage (n/(n+m)) |
|---|---|---|---|
| 1 | 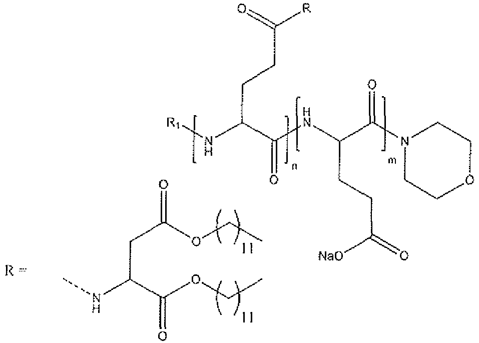 | 40 | 9 % |
| 2 | 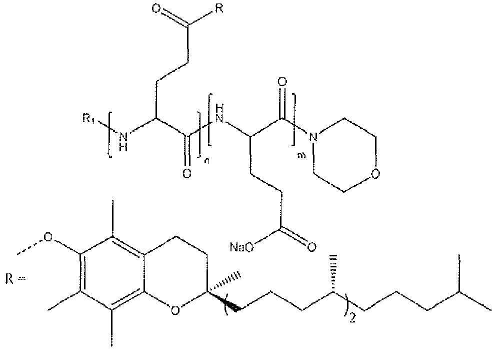 | 200 | 10 % |
| 5 | | 20 | 5 % |
| 7 | | 200 | 5 % |
| 3 | 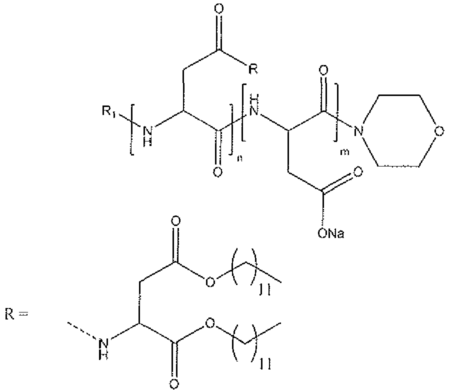 | 20 | 4 % |
| 4 | 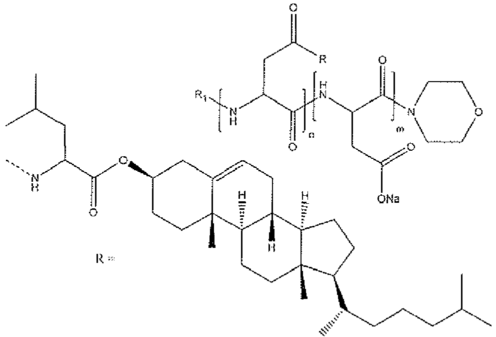 | 20 | 3 % |
| 6 | 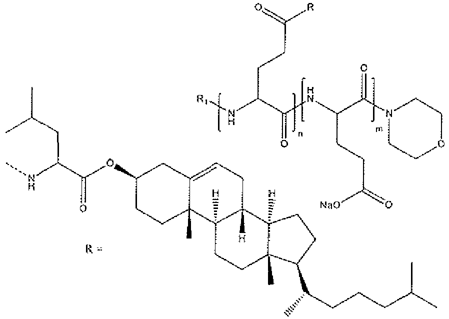 | 20 | 1,5 % |
| 8 | 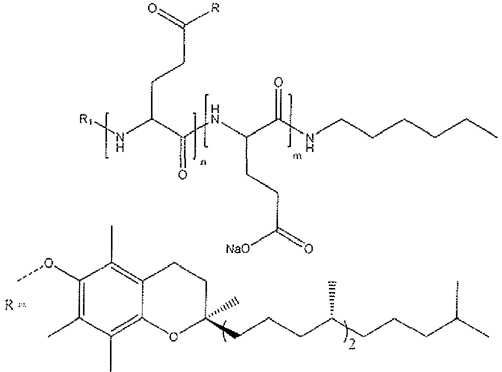 | 200 | 5 % |

### Exemple 1 : co-Polyglutamate de sodium modifié par l'aspartate de dilauryle

### Co-Polyaminoacide 1

Un polymère d'acide poly-γ-benzyl-L-glutamique de degré de polymérisation d'environ 40 est synthétisé à partir du γ-benzyl N-carboxyanhydride d'acide glutamique selon le procédé décrit dans la publication J. Am. Chem. Soc. 2008, 130, 12562-12563 (Lu H., et al.) en utilisant la N-triméthylsilyle-morpholine comme initiateur. Le degré de polymérisation est estimé par RMN ¹H en comparant l'intégration des signaux du bout de chaîne provenant de l'initiateur à celle des signaux provenant de l'unité répétée. Le degré de polymérisation moyen est de 40.

Pour l'hydrolyse des esters benzyliques, 10 g de polymère sont chauffés à reflux pendant 16h dans un mélange THF/MeOH en présence de soude 1N. Après retour à température ambiante, un solide blanc est isolé par filtration et analysé par RMN ¹H. Le solide obtenu est dissous dans l'eau, acidifié sur une résine Purolite anionique puis lyophilisé afin de générer l'acide poly-L-glutamique correspondant. L'aspartate de dilauryle, sel d'acide paratoluènesulfonique est préparé selon le procédé décrit dans le brevet US 4,826,818 (Kenji M., et al.).

2 g d'acide poly-L-glutamique (15,5 mmol de monomère) sont solubilisés dans le DMF puis refroidis à 0°C. 0,5 g (0,8 mmol) d'aspartate de dilauryle, sel d'acide paratoluènesulfonique est mis en suspension dans du DMF. 0,08 g (0,8 mmol) de triéthylamine est ensuite ajouté à cette suspension. Une fois la solution de polymère à 0°C, 1,73 g (17,1 mmol) de NMM et 1,85 g (17,1 mmol) de EtOCOCl sont ajoutés. Après 10 min de réaction, la solution d'aspartate de dilauryle est ajoutée et le milieu maintenu à 10°C durant 45 minutes. Le milieu est ensuite progressivement chauffé à 50°C. A 30°C, 40 mL d'une solution aqueuse d'imidazole à 100 g/L et 25 mL d'eau sont ajoutés. Après 1h30 d'agitation, la solution obtenue est ultrafiltrée sur membrane PES 5 kD contre une solution de NaCl 0,9%, de la soude 0,01N et de l'eau. La solution est lyophilisée et une analyse RMN ¹H dans l'acide trifluoroacétique deuteré est effectuée pour déterminer le taux de fonctions acides converties en amide d'aspartate de dilauryle.

D'après la RMN ¹H : le taux de greffage molaire par l'aspartate de dilauryle des acides par monomère est de 9 %.

### Exemple 2 : co-Polyglutamate de sodium modifié par l'alpha-tocophérol

### Co-Polyaminoacide 2

2 g d'acide poly-L-glutamique (15,5 mmol de monomère) de degré de polymérisation d'environ 200 sont synthétisés par un procédé similaire à celui décrit à l'exemple 1.

L'acide poly-L-glutamique est modifié par l'alpha-tocophérol (SIGMA) selon le procédé décrit dans le brevet FR 2,840,614 (Ping, C.U. ; et al.).

La solution de poly-L-glutamate de sodium modifié par l'alpha-tocophérol obtenue est lyophilisée et une analyse RMN ¹H dans l'acide trifluoroacétique deutéré est effectuée pour déterminer le taux de fonctions acides converties en ester d'alpha-tocophérol.

D'après la RMN ¹H : le taux de greffage molaire par l'alpha-tocophérol des acides par monomère est de 10 %.

### Exemple 3 : co-Polyaspartate de sodium modifié par l'aspartate de dilauryle

### Co-Polyaminoacide 3

L'aspartate de dilauryle, sel d'acide paratoluènesulfonique est préparé selon le procédé décrit dans le brevet US 4,826,818 (Kenji M., et al.).

2 g d'acide poly-L-aspartique (15,5 mmol de monomère) de degré de polymérisation d'environ 20 sont synthétisés par un procédé similaire à celui décrit dans l'exemple 1.

L'acide poly-L-aspartique est modifié par l'aspartate de dilauryle selon le procédé décrit dans l'exemple 1.

La solution de polyaspartate de sodium modifié par l'aspartate de dilauryle obtenue est lyophilisée et une analyse RMN ¹H dans l'eau deutérée est effectuée pour déterminer le taux de fonctions acides converties en amide d'aspartate de dilauryle.

D'après la RMN ¹H : le taux de greffage molaire par l'aspartate de dilauryle des acides par monomère est de 4 %.

### Exemple 4 : co-Polyaspartate de sodium modifié par le leucinate de cholestérol

### Co-Polyaminoacide 4

Le leucinate de cholestérol, sel d'acide paratoluènesulfonique est préparé selon le procédé décrit dans le brevet US 4,826,818 (Kenji M., et al.).

2 g d'acide poly-L-aspartique (15,5 mmol de monomère) de degré de polymérisation d'environ 20 sont synthétisés par un procédé similaire à celui décrit dans l'exemple 1.

L'acide poly-L-aspartique est modifié par le leucinate de cholestérol par un procédé similaire à celui décrit dans l'exemple 1.

La solution de poly-L-aspartate de sodium modifié par le leucinate de cholestérol obtenue est lyophilisée et une analyse RMN ¹H dans l'eau deutérée est effectuée pour déterminer le taux de fonctions acides converties en amide de leucinate de cholestérol.

D'après la RMN ¹H : le taux de greffage molaire par le leucinate de chlolestérol des acides par monomère est de 3 %.

### Exemple 5 : co-Polyglutamate de sodium modifié par l'alpha-tocophérol

### Co-Polyaminoacide 5

2 g d'acide poly-L-glutamique (15,5 mmol de monomère) de degré de polymérisation d'environ 20 sont synthétisés par un procédé similaire à celui décrit à l'exemple 1.

L'acide poly-L-glutamique est modifié par l'alpha-tocophérol (SIGMA) selon le procédé décrit dans le brevet FR 2,840,614 (Ping, C.U. ; et al.).

La solution de poly-L-glutamate de sodium modifié par l'alpha-tocophérol obtenue est lyophilisée et une analyse RMN ¹H dans l'acide trifluoroacétique deutéré est effectuée pour déterminer le taux de fonctions acides converties en ester d'alpha-tocophérol,

D'après la RMN ¹H : le taux de greffage molaire par l'alpha-tocophérol des acides par monomère est de 5 %.

### Exemple 6 : co-Polyglutamate de sodium modifié par le leucinate de cholestérol

### Co-Polyaminoacide 6

Le leucinate de cholestérol, sel d'acide paratoluènesulfonique est préparé selon le procédé décrit dans le brevet US 4,826,818 (Kenji M., et al.).

2 g d'acide poly-L-glutamique (15,5 mmol de monomère) de degré de polymérisation d'environ 20 sont synthétisés par un procédé similaire à celui décrit à l'exemple 1.

L'acide poly-L-glutamique est modifié par le leucinate de cholestérol préalablement neutralisé par la triéthylamine selon le procédé décrit dans le brevet FR 2,840,614 (Ping, C.U. ; et al.).

La solution de poly-L-glutamate de sodium modifié par le leucinate de cholestérol obtenue est lyophilisée et une analyse RMN ¹H dans l'acide trifluoroacétique deutéré est effectuée pour déterminer le taux de fonctions acides converties en ester d'alpha-tocophérol,

D'après la RMN ¹H : le taux de greffage molaire par le leucinate de cholestérol des acides par monomère est de 1,5 %.

### Exemple 7 : co-Polyglutamate de sodium modifié par l'alpha-tocophérol

### Co-Polyaminoacide 7

2 g d'acide poly-L-glutamique (15,5 mmol de monomère) de degré de polymérisation d'environ 200 sont synthétisés par un procédé similaire à celui décrit à l'exemple 1.

L'acide poly-L-glutamique est modifié par l'alpha-tocophérol (SIGMA) selon le procédé décrit dans le brevet FR 2,840,614 (Ping, C.U. ; et al.).

La solution de poly-L-glutamate de sodium modifié par l'alpha-tocophérol obtenue est lyophilisée et une analyse RMN ¹H dans le trifluoroacétique acide deutéré est effectuée pour déterminer le taux de fonctions acides converties en ester d'alpha-tocophérol.

D'après la RMN ¹H : le taux de greffage molaire par l'alpha-tocophérol des acides par monomère est de 5 %.

### Exemple 8 : co-Polyglutamate de sodium modifié par l'alpha-tocophérol

### Co-Polyaminoacide 8

Un polymère d'acide poly-L-glutamique de degré de polymérisation d'environ 200 est synthétisé à partir du γ-benzyl N-carboxyanhydride d'acide glutamique en utilisant l'hexylamine comme initiateur selon le procédé décrit dans le brevet FR 2, 801,226 (Thouraud, F. ; et al.).

Le poly-L-glutamate de sodium modifié par l'alpha-tocophérol (SIGMA) est obtenu selon le procédé décrit dans le brevet FR 2,840,614 (Ping, C.U. ; et al.).

La solution de poly-L-glutamate de sodium modifié par l'alpha-tocophérol obtenue est lyophilisée et une analyse RMN ¹H dans l'acide trifluoroacétique deutéré est effectuée pour déterminer le taux de fonctions acides converties en ester d'alpha-tocophérol.

D'après la RMN ¹H : le taux de greffage molaire par l'alpha-tocophérol des acides par monomère est de 5 %.

### Partie B Mise en évidence des propriétés des compositions selon l'invention

### Exemple B1 : Solution d'insuline analogue rapide (NovoLog®) à 100 UI/mL

Cette solution est une solution commerciale d'insuline aspart commercialisée par la société NOVO NORDISK sous le nom de NovoLog® aux Etats-Unis d'Amérique et Novorapid® en Europe. Ce produit est une insuline analogue rapide.

### Exemple B2 : Solution d'insuline analogue rapide (Humalog®) à 100 UI/mL

Cette solution est une solution commerciale d'insuline lispro commercialisée par la société ELI LILLY sous le nom de Humalog®. Ce produit est une insuline analogue rapide.

### Exemple B3 : Solution d'insuline analogue rapide (Apidra®) à 100 UI/mL

Cette solution est une solution commerciale d'insuline glulisine commercialisée par la société SANOFI-AVENTIS sous le nom d'Apidra®. Ce produit est une insuline analogue rapide.

### Exemple B4 : Solution d'insuline analogue lente (Lantus®) à 100 UI/mL

Cette solution est une solution commerciale d'insuline glargine commercialisée par la société SANOFI-AVENTIS sous le nom de Lantus®. Ce produit est une insuline analogue lente.

### Exemple B5 : Solution d'insuline humaine (ActRapid®) à 100 UI/mL

Cette solution est une solution commerciale d'insuline humaine de NOVO NORDISK vendue sous le nom d'ActRapid®. Ce produit est une insuline humaine.

### Exemple B6 : Solubilisation de l'insuline glargine à 100 UI/mL et à pH 7 à l'aide d'un co-polyaminoacide substitué à la concentration de 10 mg/mL

20 mg d'un co-polyaminoacide substitué choisi parmi ceux décrits dans le tableau 1 sont pesés précisément. Ce lyophilisat est repris par 2 mL de la solution d'insuline glargine de l'exemple B4 afin d'obtenir une solution dont la concentration en co-polyaminoacide substitué est égale à 10 mg/mL comme décrit dans le tableau 2. Après agitation mécanique sur rouleaux à température ambiante, la solution devient limpide. Le pH de cette solution est de 6,3. Le pH est ajusté à 7 avec une solution de soude 0,1 N. Cette solution limpide est filtrée sur membrane (0,22 µm) puis est placée à +4°C.

L'essai de solubilisation selon le protocole ci-dessus a été réalisé avec les co-polyaminoacides 1, 2, 3, 4, 5, 6, 7 et 8. Ces solutions sont référencées dans le Tableau 2.

**Tableau 2 : Solutions selon l'exemple B6 avec les co-polyaminoacides 1, 2, 3, 4, 5, 6, 7 et 8 de concentration 10 mg/mL**

| Solution exemple B6 | Co-polyaminoacide substitué | Concentration en co-polyaminoacide substitué |
|---|---|---|
| B6(a) | 1 | 10 mg/mL |
| B6(b) | 2 | 10 mg/mL |
| B6(c) | 3 | 10 mg/mL |
| B6(d) | 4 | 10 mg/mL |
| B6(e) | 5 | 10 mg/mL |
| B6(f) | 6 | 10 mg/mL |
| B6(g) | 7 | 10 mg/mL |
| B6(h) | 8 | 10 mg/mL |

Généralisation : Des solutions limpides d'insuline glargine à 100 UI/mL et à pH 7 ont également été obtenues avec des concentrations en co-polyaminoacides substitués de 20, 40 ou 60 mg/mL en suivant le même protocole que celui décrit dans l'exemple B6. Ainsi, une masse de co-polyaminoacide substitué lyophilisé parmi ceux décrits dans le tableau 1 est pesée précisément. Ce lyophilisat est repris par la solution d'insuline glargine de l'exemple B4 de manière à obtenir une solution dont la concentration en co-polyaminoacide substitué est de 20, 40 ou 60 mg/mL comme décrit dans le tableau 3. Après agitation mécanique sur rouleaux à température ambiante, la solution devient limpide. Le pH de cette solution est inférieur à 7. Le pH est ensuite ajusté à 7 avec une solution de soude 0,1 N. Cette solution finale limpide est filtrée sur membrane (0,22 µm) puis est placée à +4°C.

**Tableau 3 : Préparation d'une solution d'insuline glargine à 100 UI/mL et à pH 7 à l'aide d'un co-polyaminoacide substitué à la concentration de 10, 20, 40 ou 60 mg/mL**

| Concentration finale en co-polyaminoacide substitué (mg/mL) | Masse de co-polyaminoacide substitué pesée (mg) | Volume de la solution d'insuline glargine de l'exemple B4 ajouté (mL) |
|---|---|---|
| 10 | 20 | 2 |
| 20 | 40 | 2 |
| 40 | 80 | 2 |
| 60 | 120 | 2 |

### Exemple B7 : Préparation d'une composition co-polyaminoacide substitué 1/insuline glargine/insuline glulisine avec un ratio insuline glargine/insuline glulisine 75/25 à pH 7.

A 0,75 mL de la solution de co-polyaminoacide substitué 1/insuline glargine préparée selon le protocole décrit à l'exemple B6(a) est ajouté 0,25 mL de la solution d'insuline glulisine de l'exemple B3 pour former 1 mL d'une composition à pH 7. La composition est limpide attestant de la bonne solubilité de l'insuline glargine et l'insuline glulisine dans ces conditions de formulation. Cette solution limpide est filtrée sur 0,22 µm puis est placée à +4°C.

### Exemple B8 : Préparation d'une composition co-polyaminoacide substitué 1/insuline glargine/insuline lispro avec un ratio insuline glargine/insuline lispro 75/25 à pH 7

A 0,75 mL de la solution de co-polyaminoacide substitué 1/insuline glargine préparée selon le protocole décrit à l'exemple B6(a) est ajouté 0,25 mL de la solution d'insuline lispro de l'exemple B2 pour former 1 mL d'une composition à pH 7. La composition est limpide attestant de la bonne solubilité de l'insuline glargine et de l'insuline lispro dans ces conditions de formulation. Cette solution limpide est filtrée sur 0,22 µm puis est placée à +4°C.

### Exemple B9 : Préparation d'une composition co-polyaminoacide substitué 1/insuline glargine/insuline aspart avec un ratio insuline glargine / insuline aspart 75/25 à pH 7

A 0,75 mL de la solution de co-polyaminoacide substitué 1/ insuline glargine préparée à l'exemple B6(a) est ajouté 0,25 mL de la solution d'insuline aspart de l'exemple B1 pour former 1 mL d'une composition à pH 7. La composition est limpide attestant de la bonne solubilité de l'insuline glargine et de l'insuline aspart dans ces conditions de formulation. Cette solution limpide est filtrée sur 0,22 µm puis est placée à +4°C.

### Exemple B10 : Préparation d'une composition co-polyaminoacide substitué 1/ insuline glargine /insuline humaine avec un ratio insuline glargine /insuline humaine 75/25 à pH 7

A 0,75 mL de la solution de co-polyaminoacide substitué 1/ insuline glargine préparée à l'exemple B6(a) est ajouté 0,25 mL de la solution d'insuline humaine de l'exemple B5 pour former 1 mL d'une composition à pH 7. La composition est limpide attestant de la bonne solubilité de l'insuline glargine et de l'insuline humaine dans ces conditions de formulation. Cette solution limpide est filtrée sur 0,22 µm puis est placée à +4°C.

### Exemple B11 : Préparation d'une composition co-polyaminoacide substitué 1/ insuline glargine / insuline glulisine avec un ratio insuline glargine / insuline glulisine 60/40 à pH 7

A 0,6 mL de la solution de co-polyaminoacide substitué 1/ insuline glargine préparée à l'exemple B6(a) est ajouté 0,4 mL de la solution d' insuline glulisine de l'exemple B3 pour former 1 mL d'une composition à pH 7. La composition est limpide attestant de la bonne solubilité de l'insuline glargine et de l'insuline glulisine dans ces conditions de formulation. Cette solution limpide est filtrée sur 0,22 µm puis est placée à +4°C.

### Exemple B12 : Préparation d'une composition co-polyaminoacide substitué 1/ insuline glargine / insuline glulisine avec un ratio insuline glargine /insuline glulisine 40/60 à pH 7

A 0,4 mL de la solution de co-polyaminoacide substitué 1/ insuline glargine préparée à l'exemple B6(a) est ajouté 0,6 mL de la solution d'insuline glulisine de l'exemple B3 pour former 1 mL d'une composition à pH 7. La composition est limpide attestant de la bonne solubilité de l'insuline glargine et de l'insuline glulisine dans ces conditions de formulation. Cette solution limpide est filtrée sur 0,22 µm puis est placée à +4°C.

### Exemple B13 : Précipitation de l'insuline glargine

1 mL de la solution d' insuline glargine de l'exemple B4 est ajouté dans 2 mL d'une solution de PBS (tampon phosphate, phosphate buffered saline) contenant 20 mg/mL de BSA (sérum albumine bovine, bovine sérum albumin). Le mélange PBS/BSA simule la composition du milieu sous-cutané. Un précipité apparaît ce qui est en bon accord avec le mécanisme de fonctionnement de l'insuline glargine (précipitation à l'injection due à l'augmentation du pH).

Une centrifugation à 4000 trs/min est effectuée pour séparer le précipité du surnageant. Ensuite l'insuline glargine est dosée dans le surnageant par RP-HPLC. Il en résulte que l'insuline glargine se retrouve majoritairement sous une forme précipitée.

### Exemple B14 : Précipitation d'une composition co-polyaminoacide substitué 1/ insuline glargine

1 mL de solution co-polyaminoacide substitué 1/ insuline glargine préparée à l'exemple B6(a) est ajouté dans 2 mL d'une solution de PBS contenant 20 mg/mL de BSA (bovine sérum albumin). Le mélange PBS/BSA simule la composition du milieu sous-cutané. Un précipité apparaît.

Une centrifugation à 4000 trs/min est effectuée pour séparer le précipité du surnageant. Ensuite l'insuline glargine est dosée dans le surnageant par RP-HPLC. Il en résulte que l'insuline glargine se retrouve majoritairement sous une forme précipitée.

Des essais de solubilisation et précipitation identiques à ceux décrits à l'exemple B6(a) et B14 ont été effectués avec d'autres co-polyaminoacides substitués à la même concentration de 10 mg/mL de co-polyaminoacide substitué pour 100 UI/mL de solution d'insuline glargine. Il en résulte que pour l'ensemble des compositions B6(b) à B6(h) l'insuline glargine se retrouve majoritairement sous une forme précipitée à l'issue de l'ajout d'1 mL de la composition à 2 mL d'une solution de PBS contenant 20 mg/mL de BSA (bovine sérum albumin). Les résultats sont résumés dans le tableau 4.

**Tableau 4 : Essais de solubilisation et de précipitation d'une composition de co-polyaminoacide substitué/insuline glargine**

| Co-Polyaminoacide substitué (10 mg/mL) | Solubilisation de l'insuline glargine | Précipitation de l'insuline glargine |
|---|---|---|
| 1 | Oui | Oui |
| 2 | Oui | Oui |
| 3 | Oui | Oui |
| 4 | Oui | Oui |
| 5 | Oui | Oui |
| 6 | Oui | Oui |
| 7 | Oui | Oui |
| 8 | Oui | Oui |

### Exemple B15 : Précipitation d'une composition co-polyaminoacide substitué 1/insuline glargine/insuline glulisine avec un ratio insuline glargine/insuline glulisine 75/25 à pH 7

1 mL de la composition co-polyaminoacide substitué 1/insuline glargine/insuline glulisine 75/25 préparée selon le protocole de l'exemple B7 est ajouté dans 2 mL d'une solution de PBS contenant 20 mg/mL de BSA (bovine sérum albumin). Le mélange PBS/BSA simule la composition du milieu sous-cutané. Un précipité apparaît.

Une centrifugation à 4000 trs/min est effectuée pour séparer le précipité du surnageant. Ensuite l'insuline glargine est dosée dans le surnageant par RP-HPLC. Il en résulte que l'insuline glargine se retrouve majoritairement sous une forme précipitée.

### Exemple B16 : Précipitation de différentes compositions en faisant varier la nature du co-polyaminoacide substitué

D'autres essais de précipitation de l'insuline glargine dans les mêmes conditions que celles de l'exemple B15 ont été effectués en présence d'autres co-polyaminoacides.

Les résultats sont regroupés dans le tableau 5 suivant et on observe que la solubilisation ainsi que la précipitation de l'insuline glargine sont conservées.

**Tableau 5 : Essais de solubilisation et de précipitation d'une composition co-polyaminoacide substitué/insuline glargine/insuline glulisine 75/25 à pH 7**

| Co-polyaminoacide substitué | Solubilisation insuline glargine/insuline glulisine 75/25 | Précipitation De l'insuline glargine |
|---|---|---|
| 1 | Oui | Oui |
| 2 | Oui | Oui |
| 3 | Oui | Oui |
| 4 | Oui | Oui |
| 5 | Oui | Oui |
| 6 | Oui | Oui |
| 7 | Oui | Oui |
| 8 | Oui | Oui |

### Exemple B17 : Précipitation de différentes compositions en faisant varier la nature de l'insuline prandiale

Des compositions sont préparées en mélangeant 0,75 mL de la solution de co-polyaminoacide substitué 1/insuline glargine préparée selon le protocole de l'exemple B6(a) avec 0,25 mL d'une insuline prandiale pour former 1 mL de composition co-polyaminoacide substitué 1/insuline glargine/insuline prandiale (contenant 7,5 mg/mL de co-polyaminoacide substitué 1, 75 UI/mL d'insuline glargine et 25 UI/mL d'insuline prandiale).

Cette composition est ajoutée dans 2 mL de PBS contenant 20 mg/mL de BSA (bovine sérum albumin). Le mélange PBS/BSA simule la composition du milieu sous-cutané. Un précipité apparaît. Une centrifugation à 4000 trs/min est effectuée pour séparer le précipité du surnageant. Ensuite l'insuline glargine est dosée dans le surnageant par RP-HPLC. Il en résulte que l'insuline glargine se retrouve majoritairement sous une forme précipitée. En présence des 4 insulines prandiales testées, l'insuline glargine précipite dans le mélange PBS/BSA. Les résultats sont regroupés dans le tableau 6.

**Tableau 6 : Essais de solubilisation et de précipitation d'une composition co-polyaminoacide substitué 1/insuline glargine/insuline prandiale 75/25**

| Nature de l'insuline prandiale | Solubilisation insuline glargine/insuline prandiale 75/25 | Précipitation de l'insuline glargine |
|---|---|---|
| insuline glulisine (Apidra®) | Oui | Oui |
| Insuline aspart (NovoLog®) | Oui | Oui |
| Insuline lispro (Humalog®) | Oui | Oui |
| Insuline humaine (ActRapid®) | Oui | Oui |

### Exemple B18 : Préparation d'une solution d'insuline analogue lente (glargine) concentrée.

Une solution commerciale d'insuline glargine commercialisée par la société SANOFI-AVENTIS sous le nom de Lantus® est concentrée par ultrafiltration sur une membrane 3 kDa en cellulose régénérée (Amicon® Ultra-15 commercialisée par la société Millipore). A l'issue de cette étape d'ultrafiltration la concentration en insuline glargine est dosée dans le rétentat par chromatographie liquide en phase inverse (RP-HPLC). La concentration finale en insuline glargine est ensuite ajustée par l'ajout de solution commerciale de glargine à 100 UI/mL pour obtenir la concentration finale souhaitée. Ce procédé permet d'obtenir des solutions concentrées de glargine notées C_{insuline glargine} à diverses concentrations supérieures à 100 UI/mL, telles que C_{insuline glargine} = 200, 250, 300 et 333 UI/mL. Les solutions concentrées sont filtrées sur 0,22 µm puis stockées à +4°C.

### Exemple B19 : Dialyse d'une solution commerciale d'insuline analogue rapide (insuline lispro).

Une solution commerciale d'insuline lispro commercialisée par la société ELI LILLY sous le nom d'Humalog® est dialysée par ultrafiltration sur une membrane 3 kDa en cellulose régénérée (Amicon® Ultra-15 commercialisée par la société Millipore). La dialyse est réalisée dans un tampon phosphate 1 mM à pH 7. A l'issue de cette étape de dialyse la concentration C_{insuline lispro} dialysé dans le rétentat est déterminée par chromatographie liquide en phase inverse (RP-HPLC). La solution dialysée est conservée dans un congélateur à -80°C.

### Exemple B20 : Lyophilisation d'une solution d'insuline analogue rapide (insuline lispro) sous sa forme commerciale.

Un volume V_{Humalog} d'une solution d'insuline rapide lispro à une concentration de 100 UI/mL dans sa forme commerciale est placé dans un lyogard® préalablement stérilisé dans un autoclave. Le lyogard® est placé dans un congélateur à -80°C pendant environ 1h puis une lyophilisation avec les paramètres de température 20°C et de pression 0,31 mbar est effectuée.

Le lyophilisat stérile ainsi obtenu est conservé à température ambiante.

### Exemple B21 : Lyophilisation d'une solution commerciale d'insuline analogue rapide (insuline lispro) dialysée.

Un volume V_{Humalog dialysé} d'une solution d'insuline rapide lispro obtenu selon l'exemple B19 à une concentration de C_{lispro dialysé} est placé dans un lyogard® préalablement stérilisé dans un autoclave. Le lyogard® est placé dans un congélateur à -80°C pendant environ 1h puis une lyophilisation avec les paramètres de température 20°C et de pression 0,31 mbar est effectuée,

Le lyophilisat stérile ainsi obtenu est conservé à température ambiante.

### Exemple B22 : Préparation d'une composition co-polyaminoacide substitué/glargine à pH 7 à l'aide du co-polyaminoacide substitué 5, suivant un procédé utilisant glargine sous forme liquide (en solution) et un co-polyaminoacide sous forme solide (lyophilisée).

Une masse m_{co-polyaminoacide} de co-polyaminoacide 5 est pesée précisément. Ce lyophilisat est repris par un volume V_{glargine} d'une solution concentrée de glargine préparée selon l'exemple B18 de manière à obtenir une composition présentant une concentration de co-polyaminoacide C_{co-polyaminoacide} (mg/mL) = m_{co-polyaminoacide}/V_{insuline glargine} et une concentration en insuline glargine C_{insuline glargine} (UI/mL). La solution est opalescente. Le pH de cette solution est d'environ 6,3. Le pH est ajusté à 7 par ajout de NaOH concentrée puis la solution est placée en statique dans une étuve à 37°C pendant environ 1 heure jusqu'à solubilisation complète. Un volume V_{co-polyaminoacide/insuline glargine} de cette solution visuellement limpide est placée à +4°C.

### Exemple B23 : Préparation d'une composition co-polyaminoacide substitué 5/glargine à pH 7 à l'aide d'un co-polyaminoacide substitué 5, suivant un procédé utilisant glargine sous forme liquide (en solution) et un co-polyaminoacide sous forme liquide (en solution).

A une solution mère de co-polyaminoacide 5 à pH 7 présentant une concentration C_{mère co-polyaminoacide} sont ajoutées des solutions concentrées de m-crésol, glycérine et Tween® 20 de manière à obtenir une solution de co-polyaminoacide de concentration C_{mère co-polyaminoacide/excipients} (mg/mL) en présence de ces excipients à des teneurs équivalentes à celles décrites dans la solution commerciale Lantus® en flacon de 10 mL.

Dans un pot stérile, un volume V_{Lantus} d'une solution commerciale d'insuline lente glargine commercialisée sous le nom de Lantus® à une concentration de 100 UI/mL est ajouté à un volume V_{mère co-polyaminoacide/excipients} d'une solution de co-polyaminoacide à la concentration C_{mère co-polyaminoacide/excipients} (mg/mL). Un trouble apparaît. Le pH est ajusté à pH 7 par ajout de NaOH concentrée et la solution est placée en statique dans une étuve à 37°C pendant environ 1h jusqu'à solubilisation complète. Cette solution visuellement limpide est placée à +4°C.

### Exemple B24 : Préparation d'une composition co-polyaminoacide 5/glargine concentrée à pH=7 à l'aide d'un co-polyaminoacide substitué 5, suivant un procédé de concentration d'une composition diluée.

Une composition co-polyaminoacide 5/glargine diluée à pH 7 décrite dans l'exemple B23 est concentrée par ultrafiltration sur une membrane 3 kDa en cellulose régénérée (Amicon® Ultra-15 commercialisée par la société Millipore). A l'issue de cette étape d'ultrafiltration, le rétentat est limpide et la concentration en insuline glargine dans la composition est déterminée par chromatographie en phase inverse (RP-HPLC). Si nécessaire, la concentration en insuline glargine dans la composition est ensuite ajustée à la valeur souhaitée par dilution dans une solution d'excipients m-crésol/Glycérine/Tween®20 présentant, pour chaque espèce une concentration équivalente à celle décrite dans la solution commerciale Lantus® (en flacon de 10 mL). Cette solution à pH 7, visuellement limpide, présentant une concentration en glargine C_{glargine} (UI/mL) et une concentration en co-polyaminoacide C_{co-polyaminoacide} (mg/mL), est placée à +4°C.

### Exemple B25 : Préparation d'une composition co-polyaminoacide substitué 5/insuline glargine/insuline lispro à pH 7, à partir d'un lyophilisat d'une insuline rapide lispro sous sa forme commerciale (Humalog®).

Un volume V_{co-polyaminoacide/glargine} de solution de co-polyaminoacide 5/glargine pH 7 présentant une concentration en glargine C_{insuline glargine} (UI/mL) et une concentration de co-polyaminoacide 5 C_{co-polyaminoacide} (mg/mL) préparé selon l'exemple B22 est ajouté sur un lyophilisat d'insuline lispro obtenu par lyophilisation d'un volume V_{insuline lispro} dont la préparation est décrite dans l'exemple B20, de telle manière que le ratio V_{co-polyaminoacide/insuline glargine} / V_{insuline lispro} = 100 / C_{insuline lispro} où C_{insuline lispro} est la concentration en insuline lispro (UI/mL) visée dans la composition. La solution est limpide. La teneur en zinc de la formulation est ajustée à la concentration C_{zinc} (µM) souhaitée par ajout d'une solution concentrée de chlorure de zinc. Le pH final est ajusté à 7 par ajout de NaOH ou HCl concentré.

La formulation est limpide, attestant de la bonne solubilité de glargine et insuline lispro dans ces conditions de formulation. Cette solution est filtrée sur 0,22 µm et placée à +4°C.

### Exemple B26 : Préparation d'une composition co-polyaminoacide substitué 5/glargine/insuline lispro à pH 7, à partir d'un lyophilisat d'une insuline rapide lispro obtenue par dialyse d'une solution commerciale (Humalog®).

Un volume V_{co-polyaminoacide/insuline glargine} de solution de co-polyaminoacide 5/glargine pH 7 présentant une concentration en glargine C_{insuline glargine} (UI/mL) et une concentration de co-polyaminoacide 5 C_{co-polyaminoacide} (mg/mL) préparé selon l'exemple B22 est ajouté sur un lyophilisat d'insuline lispro obtenu par lyophilisation d'un volume V_{insuline lispro dialysé} dont la préparation est décrite dans l'exemple B21, de telle manière que le ratio V_{co-polyaminoacide/insuline glargine}/V_{insuline lispro dialysé} = C_{insuline lispro dialysé}/C_{insuline lispro} où C_{insuline lispro dialysé} est la concentration en insuline lispro (UI/mL) obtenue à l'issue de la dialyse de la solution commerciale, étape décrite dans l'exemple B19, et C_{insuline lispro} est la concentration en insuline lispro (UI/mL) visée dans la composition. La solution est limpide. La teneur en zinc de la formulation est ajustée à la concentration C_{zinc} (µM) souhaitée par ajout d'une solution concentrée de chlorure de zinc. Le pH final est ajusté à 7 par ajout de NaOH ou HCl concentré.

La formulation est limpide, attestant de la bonne solubilité des insulines glargine et lispro dans ces conditions de formulation. Cette solution est filtrée sur 0,22 µm et placée à +4°C.

### Exemple B27 : Préparation d'une composition co-polyaminoacide substitué 5/insuline glargine/insuline lispro à pH 7 présentant une concentration en glargine de 200 UI/mL et une concentration de insuline lispro de 66 UI/mL (proportion en pourcentage d'insuline : 75/25 en glargine/insuline lispro).

Une solution d'insuline glargine concentrée à 200 UI/mL est préparée selon l'exemple B18. Une composition co-polyaminoacide 5 (20 mg/mL)/insuline glargine 200 UI/mL pH 7 est préparée à partir du co-polyaminoacide 5 et selon le mode de préparation décrit à l'exemple B22. Cette composition co-polyaminoacide 5 /insuline glargine 200 UI/mL est ajoutée à un lyophilisat d'insuline lispro obtenu par lyophilisation de la solution d'analogue rapide sous sa forme commerciale, selon le mode de préparation décrit dans l'exemple B25. La solution est limpide. La teneur en zinc de la formulation est ajustée à la concentration souhaitée par ajout d'une solution concentrée de chlorure de zinc. Le pH final est ajusté à 7 par ajout de NaOH ou HCl concentré.

La formulation est limpide, attestant de la bonne solubilité des insulines glargine et lispro dans ces conditions de formulation. Cette solution est filtrée sur 0,22 µm et placée à +4°C.

Cette composition est décrite dans le tableau 11.

Des compositions co-polyaminoacide substitué/insuline glargine/insuline lispro à pH 7 ont également été préparées avec d'autres co-polyaminoacides selon un mode de préparation identique à celui décrit dans l'exemple B27 avec une concentration en co-polyaminoacide substitué d'au plus 40 mg/mL. Ces formulations sont limpides, attestant de la bonne solubilité des insulines glargine et lispro dans ces conditions de formulation. Ces compositions conduisent aux exemples répertoriés dans le tableau 7.

**Tableau 7**

| Exemple | Co-Polyaminoacide substitué |
|---|---|
| B28 | 3 |
| B29 | 4 |
| B30 | 7 |
| B31 | 8 |

### Exemple B32 : Préparation d'une composition co-polyaminoacide substitué 5/insuline glargine/insuline lispro à pH 7 présentant une concentration en glargine de 300 UI/mL et une concentration en insuline lispro de 100 UI/mL (proportion en pourcentage d'insuline : 75/25 en insuline glargine/insuline lispro).

Une solution d'insuline glargine concentrée à 300 UI/mL est préparée selon l'exemple B18. Une composition co-polyaminoacide 5 (10 mg/mL)/insuline glargine 300 UI/mL pH 7 est préparée à partir du co-polyaminoacide 5 et selon le mode de préparation décrit à l'exemple B22. Cette composition co-polyaminoacide 5/glargine 300 UI/mL est ajoutée à un lyophilisat d'insuline lispro obtenu par lyophilisation de la solution d'analogue rapide sous sa forme commerciale, selon le mode de préparation décrit dans l'exemple B25. La solution est limpide. La teneur en zinc de la formulation est ajustée à la concentration souhaitée par ajout d'une solution concentrée de chlorure de zinc. Le pH final est ajusté à 7 par ajout de NaOH ou HCl concentré.

La formulation est limpide, attestant de la bonne solubilité des insulines glargine et lispro dans ces conditions de formulation. Cette solution est filtrée sur 0,22 µm et placée à +4°C. Cette composition est décrite dans le tableau 7.

Des compositions co-polyaminoacide substitué/insuline glargine/insuline lispro à pH 7 ont également été préparées avec d'autres co-polyaminoacides selon un mode de préparation identique à celui décrit dans l'exemple B32 avec une concentration en co-polyaminoacide substitué d'au plus 40 mg/mL. Ces formulations sont limpides, attestant de la bonne solubilité des insulines glargine et lispro dans ces conditions de formulation. Ces compositions conduisent aux exemples répertoriés dans le tableau 8.

**Tableau 8**

| Exemple | Polyaminoacide substitué |
|---|---|
| B33 | 2 |
| B34 | 3 |
| B35 | 7 |
| B36 | 8 |

### Exemple B37 : Préparation d'une composition co-polyaminoacide substitué 5 /insuline glargine/insuline lispro à pH 7 présentant une concentration en insuline glargine de 250 UI/mL et une concentration en insuline lispro de 150 UI/mL (proportion en pourcentage d'insuline : 63/37 en insuline glargine/insuline lispro).

Une solution d'insuline glargine concentrée à 250 UI/mL est préparée selon l'exemple B18. Une composition co-polyaminoacide 5 (25 mg/mL)/glargine 250 UI/mL pH 7 est préparée à partir du co-polyaminoacide 5 et selon le mode de préparation décrit à l'exemple B22. Cette composition co-polyaminoacide 5 /glargine 250 UI/mL est ajoutée à un lyophilisat d'insuline lispro obtenu par lyophilisation de la solution d'analogue rapide sous sa forme commerciale, selon le mode de préparation décrit dans l'exemple B25. La solution est limpide. La teneur en zinc de la formulation est ajustée à la concentration souhaitée par ajout d'une solution concentrée de chlorure de zinc. Le pH final est ajusté à 7 par ajout de NaOH ou HCl concentré.

La formulation est limpide, attestant de la bonne solubilité des insulines glargine et lispro dans ces conditions de formulation. Cette solution est filtrée sur 0,22 µm et placée à +4°C.

Cette composition est décrite dans le tableau 11.

Des compositions co-polyaminoacide substitué/insuline glargine/insuline lispro à pH 7 ont également été préparées avec d'autres co-polyaminoacides selon un mode de préparation identique à celui décrit dans l'exemple B37 avec une concentration en co-polyaminoacide substitué d'au plus 40 mg/mL. Ces formulations sont limpides, attestant de la bonne solubilité des insulines glargine et lispro dans ces conditions de formulation. Ces compositions conduisent aux exemples répertoriés dans le tableau 9.

**Tableau 9**

| Exemple | Polyaminoacide substitué |
|---|---|
| B38 | 3 |
| B39 | 7 |
| B40 | 8 |

### Exemple B41 : Préparation d'une composition co-polyaminoacide substitué 5/insuline glargine/insuline lispro à pH 7 présentant une concentration en insuline glargine de 333 UI/mL et une concentration en insuline lispro de 67 UI/mL (proportion en pourcentage d'insuline : 83/17 en insuline glargine/insuline lispro).

Une solution d'insuline glargine concentrée à 333 UI/mL est préparée selon l'exemple B18. Une composition co-polyaminoacide 5 (33 mg/mL)/insuline glargine 300 UI/mL pH 7 est préparée à partir du co-polyaminoacide 5 et selon le mode de préparation décrit à l'exemple B22. Cette composition co-polyaminoacide 5 /insuline glargine 333 UI/mL est ajoutée à un lyophilisat d'insuline lispro obtenu par lyophilisation de la solution d'analogue rapide sous sa forme commerciale, selon le mode de préparation décrit dans l'exemple B25. La solution est limpide. La teneur en zinc de la formulation est ajustée à la concentration souhaitée par ajout d'une solution concentrée de chlorure de zinc. Le pH final est ajusté à 7 par ajout de NaOH ou HCl concentré.

La formulation est limpide, attestant de la bonne solubilité des insulines glargine et lispro dans ces conditions de formulation. Cette solution est filtrée sur 0,22 µm et placée à +4°C.

Cette composition est décrite dans le tableau 11.

Des compositions co-polyaminoacide substitué/insuline glargine/insuline lispro à pH 7 ont également été préparées avec d'autres co-polyaminoacides selon un mode de préparation identique à celui décrit dans l'exemple B41 avec une concentration en co-polyaminoacide substitué d'au plus 40 mg/mL. Ces formulations sont limpides, attestant de la bonne solubilité des insulines glargine et lispro dans ces conditions de formulation. Ces compositions conduisent aux exemples répertoriés dans le tableau 10.

**Tableau 10**

| Exemple | Polyaminoacide substitué |
|---|---|
| B42 | 3 |
| B43 | 7 |
| B44 | 8 |

### Exemple B45 : Précipitation de différentes compositions co-polyaminoacide substitué/insuline glargine/insuline lispro à pH 7 présentant différentes concentrations en insulines glargine et lispro et différentes proportions relatives des 2 insulines.

1 mL de composition co-polyaminoacide substitué/ insuline glargine/insuline lispro préparée aux exemples B27 à B44 est ajouté dans 2 mL d'une solution de PBS contenant 20 mg/mL de BSA (bovine serum albumin). Le mélange PBS/BSA simule la composition du milieu sous-cutané. Un précipité apparaît. Une centrifugation à 4000 trs/min est effectuée pour séparer le précipité du surnageant. Ensuite l'insuline glargine est dosée dans le surnageant par RP-HPLC. Il en résulte que l'insuline glargine se retrouve majoritairement sous une forme précipitée.

Les résultats de solubilisation et de précipitation sont résumés dans le tableau 11.

**Tableau 11 : Essais de solubilisation et de précipitation de différentes compositions co-polyaminoacide substitué/ insuline glargine/insuline lispro à pH 7 présentant différentes concentrations en insuline glargine et lispro et différentes proportions relatives des 2 insulines.**

| Exemple | Polyaminoacide substitué | C_{insuline glargine} (UI/mL) | C_{insuline lispro} (UI/mL) | C_{insuline glargine}/C_{insuline lispro} (%/%) | Solubilisation insuline glargine et insuline lispro à pH 7 | Precipitation de l'insuline glargine |
|---|---|---|---|---|---|---|
| B28 | 3 | 200 | 66 | 75/25 | OUI | OUI |
| B29 | 4 | 200 | 66 | 75/25 | OUI | OUI |
| B27 | 5 | 200 | 66 | 75/25 | OUI | OUI |
| B30 | 7 | 200 | 66 | 75/25 | OUI | OUI |
| B31 | 8 | 200 | 66 | 75/25 | OUI | OUI |
| B33 | 2 | 300 | 100 | 75/25 | OUI | OUI |
| B34 | 3 | 300 | 100 | 75/25 | OUI | OUI |
| B32 | 5 | 300 | 100 | 75/25 | OUI | OUI |
| B35 | 7 | 300 | 100 | 75/25 | OUI | OUI |
| B36 | 8 | 300 | 100 | 75/25 | OUI | OUI |
| B38 | 3 | 250 | 150 | 63/37 | OUI | OUI |
| B37 | 5 | 250 | 150 | 63/37 | OUI | OUI |
| B39 | 7 | 250 | 150 | 63/37 | OUI | OUI |
| B40 | 8 | 250 | 150 | 63/37 | OUI | OUI |
| B41 | 5 | 333 | 67 | 83/17 | OUI | OUI |
| B42 | 3 | 333 | 67 | 83/17 | OUI | OUI |
| B43 | 7 | 333 | 67 | 83/17 | OUI | OUI |
| B44 | 8 | 333 | 67 | 83/17 | OUI | OUI |

### Partie C : Mise en évidence des propriétés des compositions comprenant un analogue ou un dérivé de GLP-1 selon l'invention.

### Exemple C1 : Solution de GLP-1 analogue exenatide (Byetta®) à 0,25 mg/mL

Cette solution est une solution d'exenatide commercialisée par la société ELI LILLY and Company sous le nom de Byetta®.

### Exemple C2 : Solution de GLP-1 dérivé liraglutide (Victoza®) à 6 mg/mL

Cette solution est une solution de liraglutide commercialisée par la société NOVO NORDISK sous le nom de Victoza®.

### Exemple C3 : Préparation d'une composition Lantus®/Byetta® 70/30 à pH 7,5

A 0,21 mL de la solution d'insuline glargine de l'exemple B4 est ajouté 0,09 mL de la solution d'exenatide de l'exemple C1 pour obtenir 0,3 mL d'une composition dont le pH est de 4,5 au mélange. La composition contenant 70 UI/mL d'insuline glargine et 0,075 mg/mL d'exenatide est limpide attestant de la bonne solubilité de l'insuline glargine et de l'exenatide dans ces conditions de formulation (pH 4,5). Le pH est ensuite ajusté à 7,5 avec une solution de soude 0,1 N. La composition devient alors trouble attestant de la mauvaise solubilité de la composition insuline glargine exenatide à pH 7,5.

Des compositions Lantus®/Byetta® 70/30 ont également été préparées à pH 4,5 - 5,5 - 6,5 - 8,5 et 9,5 en suivant un protocole similaire à celui décrit dans l'exemple C3. Pour chacune de ces compositions, à 0,21 mL de la solution d'insuline glargine de l'exemple B4 est ajouté 0,09 mL de la solution d'exenatide de l'exemple C1 pour obtenir 0,3 mL d'une composition dont le pH est de 4,5 au mélange. La composition est limpide attestant de la bonne solubilité de l'insuline glargine et de l'exenatide dans ces conditions de formulation (pH 4,5). Le pH est ajusté à 5,5 ou 6,5 ou 8,5 ou 9,5 avec une solution de soude 0,1 N. Après ajustement du pH, la composition à pH 5,5 est légèrement trouble, les compositions à pH 6,5 et 8,5 sont très troubles et la composition à pH 9,5 est limpide. Ces compositions sont placées à +4°C pendant 48h. Après 48h à +4°C, seule la composition à pH 4,5 reste limpide. L'aspect visuel après 48h des compositions Lantus®/Byetta®70/30 à différents pH est résumé dans le tableau 12.

**Tableau 12 : Aspect visuel après 48h à 4°C des compositions Lantus®/Byetta® 70/30 à différents pH**

| Compositions Lantus®/Byetta® 70/30 à différents pH | |
|---|---|
| pH | Aspect Visuel à t = 48h |
| 4,5 | Limpide |
| 5,5 | Présence d'un précipité |
| 6,5 | Présence d'un précipité |
| 7,5 | Présence d'un précipité |
| 8,5 | Présence d'un précipité |
| 9,5 | Présence d'un précipité |

### Exemple C4 : Préparation d'une composition Lantus®/Victoza® 70/30 à pH 7,5

A 0,21 mL de la solution d'insuline glargine de l'exemple B4 est ajouté 0,09 mL de la solution de liraglutide de l'exemple C2 pour obtenir 0,3 mL d'une composition dont le pH est de 7 au mélange. La composition contenant 70 UI/mL d'insuline glargine et 1,8 mg/mL de liraglutide est trouble attestant de la mauvaise solubilité de la composition insuline glargine /liraglutide dans ces conditions de formulation. Le pH est ajusté à 7,5 avec une solution de soude 0,1 N. Après ajustement du pH, la composition reste trouble. Cette composition est placée à +4°C pendant 48h.

Des compositions Lantus®/Victoza® 70/30 ont également été préparées à pH 4,5, 5,5, 6,5, 8,5 et 9,5 en suivant un protocole similaire à celui décrit dans l'exemple C4. Pour chacune de ces compositions, à 0,21 mL de la solution d'insuline glargine de l'exemple B4 sont ajoutés 0,09 mL de la solution de liraglutide de l'exemple C2 pour obtenir 0,3 mL d'une composition dont le pH est de 7. La composition est trouble attestant de la mauvaise solubilité de la composition insuline glargine / liraglutide dans ces conditions de formulation (pH 7). Le pH est ajusté à 4,5 ou 5,5 ou 6,5 avec une solution d'acide chlorhydrique 0,1 N ou à pH 9,5 avec une solution de soude 0,1 N. Après ajustement du pH, les compositions à pH 4,5 - 5,5 - 6,5 et 8,5 sont troubles, attestant de la mauvaise solubilité de la composition insuline glargine / liraglutide dans ces conditions de formulation. Ces compositions sont placées à +4°C pendant 48h. Après 48h à 4°C, seule la composition à pH 9,5 est limpide. L'aspect visuel après 48h des compositions Lantus®/Victoza® 70/30 à différents pH est résumé dans le tableau 13.

**Tableau 13 : Aspect visuel après 48h à 4°C des compositions Lantus®/Victoza® 70/30 à différents pH**

| Compositions Lantus®/Victoza® 70/30 à différents pH | |
|---|---|
| pH | Aspect Visuel à t = 48h |
| 4,5 | Présence d'un précipité |
| 5,5 | Présence d'un précipité |
| 6,5 | Présence d'un précipité |
| 7,5 | Présence d'un précipité |
| 8,5 | Présence d'un précipité |
| 9,5 | Limpide |

### Exemple C5 : Préparation d'une composition co-polyaminoacide 1 substitué/ Lantus®/ Byetta® avec un ratio Lantus®/Byetta® 70/30 à pH 7

A 0,21 mL de la solution de co-polyaminoacide substitué 1/ insuline glargine préparée selon le protocole de l'exemple B6(a) est ajouté 0,09 mL de la solution d'exenatide de l'exemple C1 pour obtenir 0,3 mL d'une composition à pH 5,3. Le pH est ajusté à 7 avec une solution de soude 0,1 N. La composition contenant 7 mg/mL de co-polyaminoacide substitué 1, 70 UI/mL d'insuline glargine et 0,075 mg/mL d'exenatide est limpide attestant de la bonne solubilité de l'insuline glargine et de l'exenatide en présence du co-polyaminoacide substitué 1 à pH 7. Cette solution limpide est placée à +4°C.

Généralisation: Des compositions co-polyaminoacide substitué/Lantus®/ Byetta® à pH 7 ont également été préparées à des ratios volumiques V_{Lantus}/V_{Byetta} de 90/10, 50/50 et 30/70 en suivant le même protocole que celui décrit dans l'exemple C5. Ainsi, à un volume V_{co-polyaminoacide/insuline glargine} de la solution de co-polyaminoacide substitué/insuline glargine préparée selon le protocole de l'exemple B6 est ajouté un volume V_{Byetta} de la solution d'exenatide de l'exemple C1 pour obtenir une composition dont le pH est ajusté à 7 avec une solution de soude 0,1 N. Les compositions obtenues (voir tableau 14) sont limpides attestant de la bonne solubilité de l'insuline glargine et de l'exenatide en présence d'un co-polyaminoacide substitué 1 à pH 7. Ces solutions limpides sont placées à +4°C.

### Exemple C6 : Préparation d'une composition co-polyaminoacide substitué 1/Lantus® /Byetta® avec un ratio Lantus®/ Byetta® 100/50 à pH 7

0,150 mL de la solution d'exenatide de l'exemple C1 sont lyophilisés, puis 0,3 mL d'une solution de co-polyaminoacide substitué/insuline glargine préparée selon le protocole de l'exemple B6(a) sont ajoutés au lyophilisat afin d'obtenir une composition dont le pH est ajusté à 7 avec une solution de soude 0,1 N. La composition contenant 10 mg/mL de co-polyaminoacide substitué 1, 100 UI/mL d'insuline glargine et 0,125 mg/mL d'exenatide est limpide attestant de la bonne solubilité de l'insuline glargine et de l'exenatide en présence du co-polyaminoacide substitué 1 à pH 7. Cette solution limpide est placée à +4°C.

**Tableau 14: Concentrations finales des compositions obtenues aux exemples C5 et C6 en insuline glargine, co-polyaminoacide substitué 1 et exenatide**

| V_{Lantus}/ V_{Byetta} | insuline glargine mg/mL | [co-polyaminoacide substitué 1] (mg/mL) | exenatide (mg/mL) |
|---|---|---|---|
| 100/50 | 3,5 | 10 | 0,125 |
| 90/10 | 3,15 | 9 | 0,025 |
| 70/30 | 2,45 | 7 | 0,075 |
| 50/50 | 1,75 | 5 | 0,125 |
| 30/70 | 1,05 | 3 | 0,175 |

### Exemple C7: Préparation d'une composition co-polyaminoacide substitué 1/Lantus®/Victoza® avec un ratio Lantus®/Victoza® 70/30 à pH 7

A 0,21 mL de la solution de co-polyaminoacide substitué/insuline glargine préparée selon le protocole de l'exemple B6(a) est ajouté 0,09 mL de la solution de liraglutide de l'exemple C2 pour obtenir 0,3 mL d'une composition à pH 7,6. Le pH est ajusté à 7 avec une solution d'acide chlorhydrique 0,1 N. La composition contenant 7 mg/mL de co-polyaminoacide substitué 1, 70 UI/mL de insuline glargine et 1,8 mg/mL de liraglutide est limpide attestant de la bonne solubilité de l'insuline glargine et du liraglutide en présence du co-polyaminoacide substitué 1 à pH 7. Cette solution limpide est placée à +4°C. La composition finale obtenue est résumée dans le Tableau 15.

Généralisation : Des compositions co-polyaminoacide substitué/Lantus® /Victoza® à pH 7 ont également été préparées à des ratios volumiques V_{Lantus/}V_{Victoza} de 90/10, 50/50 et 30/70 en suivant le même protocole que celui décrit dans l'exemple C7. Ainsi, à un volume V_{co-polyaminoacide/insuline glargine} de la solution de co-polyaminoacide substitué 1/insuline glargine à une concentration de co-polyaminoacide de 40 mg/mL préparée selon le protocole de l'exemple B6 est ajouté un volume V_{Victoza} de la solution de liraglutide de l'exemple C2 pour obtenir une composition dont le pH est ajusté à 7 avec une solution d'acide chlorhydrique 0,1 N.

Les compositions obtenues (voir tableau 15) sont limpides attestant de la bonne solubilité de l'insuline glargine et du liraglutide en présence d'un co-polyaminoacide substitué à pH 7. Ces solutions limpides sont placées à +4°C.

**Tableau 15 : Concentrations finales des compositions obtenues à l'exemple C7 en insuline glargine, co-polyaminoacide substitué 1 et liraglutide**

| V_{Lantus}/V_{Victoza} | insuline glargine mg/mL | [co-polyaminoacide substitué 1] (mg/mL) | liraglutide (mg/mL) |
|---|---|---|---|
| 90/10 | 3,15 | 36 | 0,6 |
| 70/30 | 2,45 | 28 | 1,8 |
| 50/50 | 1,75 | 20 | 3 |
| 30/70 | 1,05 | 12 | 4,2 |

### Exemple C8: Préparation d'une composition co-polyaminoacide substitué 1/Lantus® /Apidra®/Byetta® avec un ratio Lantus®/Apidra®/Byetta® 60/20/20 à pH 7

20 mg de co-polyaminoacide substitué 1 lyophilisé sont pesés précisément. Ce lyophilisat est repris par 2 mL de la solution d'insuline glargine de l'exemple B4. Après agitation mécanique sur rouleaux à température ambiante, la solution devient limpide. Le pH de cette solution est de 6,3. Le pH est ajusté à 7 avec une solution de soude 0,1 N. A 0,6 mL de la solution co-polyaminoacide substitué/insuline glargine préparée précédemment sont ajoutés 0,2 mL de la solution d'exenatide de l'exemple C1 et 0,2 mL de la solution d'insuline glulisine de l'exemple B3 pour obtenir 1 mL d'une composition à pH 7. La composition contenant 6 mg/mL de co-polyaminoacide substitué 1, 60 UI/mL de insuline glargine, 20 UI/mL de glulisine et 0,05 mg/mL d'exenatide est limpide attestant de la bonne solubilité de l'insuline glargine, de l'insuline glulisine et de l'exenatide en présence du co-polyaminoacide substitué 1 à pH 7. Cette solution limpide est filtrée sur membrane (0,22 µm) puis est placée à +4°C.

### Exemple C9 : Précipitation d'une composition co-polyaminoacide substitué/Lantus® /Byetta® avec un ratio Lantus®/Byetta® 70/30 à pH 7

0,250 mL de la composition co-polyaminoacide substitué/Lantus®/Byetta® préparée selon le protocole de l'exemple C5 est ajouté dans 0,5 mL d'une solution de PBS contenant 20 mg/mL de BSA. Le mélange PBS/BSA simule la composition du milieu sous-cutané. Un précipité apparaît.

Une centrifugation à 4000 trs/min est effectuée pour séparer le précipité du surnageant. Ensuite l'insuline glargine est dosée dans le surnageant par RP-HPLC. L'insuline glargine se retrouve majoritairement sous une forme précipitée. Le résultat est résumé dans le Tableau 16.

### Exemple C10 : Précipitation d'une composition co-polyaminoacide substitué/Lantus® /Victoza® avec un ratio Lantus®/Victoza® 70/30 à pH 7

0,250 mL de la composition co-polyaminoacide substitué 1/Lantus® /Victoza® préparée selon le protocole de l'exemple C7 est ajouté dans 0,5 mL d'une solution de PBS contenant 20 mg/mL de BSA (bovine serum albumin). Le mélange PBS/BSA simule la composition du milieu sous-cutané. Un précipité apparaît.

Une centrifugation à 4000 trs/min est effectuée pour séparer le précipité du surnageant. Ensuite l'insuline glargine est dosée dans le surnageant par RP-HPLC. L'insuline glargine se retrouve majoritairement sous une forme précipitée. Le résultat est présenté dans le Tableau 17.

### Exemple C11 : Précipitation de différentes compositions en faisant varier la nature du co-polyaminoacide substitué

D'autres essais dans les mêmes conditions que ceux décrits dans les exemples C9 et C10 ont été effectués en présence d'autres co-polyaminoacides substitués et sont respectivements présentés dans les Tableaux 16 et 17.

Des résultats avec au plus 10 mg/mL de co-polyaminoacide substitué et une composition Lantus®/Byetta® 70/30 à pH 7 sont regroupés dans le tableau 16 suivant. On observe que la solubilisation et la précipitation de l'insuline glargine sont conservées.

**Tableaux 16 : Résultats des essais de solubilisation et de précipitation obtenus avec au plus 42 ou 10 mg/mL de co-polyaminoacide substitué et une composition Lantus®/Byetta® 70/30 à pH 7**

| Co-polyaminoacide substitué (au plus 42 mg/mL) | Solubilisation Lantus®/Byetta® 70/30 à pH 7 | Precipitation de l'insuline glargine |
|---|---|---|
| 8 | Oui | Oui |

| Co-polyaminoacide substitué (au plus 10 mg/mL) | Solubilisation Lantus®/Byetta® 70/30 à pH 7 | Precipitation de l'insuline glargine |
|---|---|---|
| 1 | Oui | Oui |
| 2 | Oui | Oui |
| 3 | Oui | Oui |
| 4 | Oui | Oui |
| 7 | Oui | Oui |
| 8 | Oui | Oui |

Des résultats avec au plus 40 mg/mL de co-polyaminoacide substitué et une composition Lantus®/Victoza® 70/30 à pH 7 sont regroupés dans le tableau 17 suivant. On observe que la solubilisation et la précipitation de de l'insuline glargine sont conservées.

**Tableaux 17 : Résultats des essais de solubilisation et de précipitation obtenus avec au plus 40 ou 42 mg/mL de co-polyaminoacide substitué et une composition Lantus®/Victoza® 70/30 à pH 7.**

| Co-polyaminoacide substitué au plus de 42 mg/mL | Solubilisation Lantus®/Victoza® 70/30 à pH 7 | Precipitation de l'insuline glargine |
|---|---|---|
| 8 | Oui | Oui |
| | | |

| Co-polyaminoacide substitué au plus de 40 mq/mL | Solubilisation Lantus®/Victoza® 70/30 à pH 7 | Precipitation de l'insuline glargine |
|---|---|---|
| 1 | Oui | Oui |
| 2 | Oui | Oui |
| 3 | Oui | Oui |
| 4 | Oui | Oui |
| 7 | Oui | Oui |
| 8 | Oui | Oui |

### Exemple C12 : Précipitation d'une composition co-polyaminoacide substitué/Lantus®/Apidra®/Byetta® 60/20/20 à pH 7

0,250 mL de la composition co-polyaminoacide substitué/Lantus® /Apidra®/Byetta® préparée à l'exemple C8 sont ajoutés dans 0,5 mL d'une solution de PBS contenant 20 mg/mL de BSA. Le mélange PBS/BSA simule la composition du milieu sous-cutané. Un précipité apparaît. Une centrifugation à 4000 trs/min est effectuée pour séparer le précipité du surnageant. Ensuite l'insuline glargine est dosée dans le surnageant par RP-HPLC. L'insuline glargine se retrouve majoritairement sous une forme précipitée.

### Partie D : Mise en évidence des propriétés pharmacodynamiques des compositions selon l'invention.

### Exemple D1 : Protocole de mesure de la Pharmacodynamie des solutions d'insuline

10 cochons domestiques d'environ 50 kg, préalablement cathétérisés au niveau de la jugulaire, sont mis à jeun 2,5 heures avant le début de l'expérience. Dans l'heure précédant l'injection d'insuline, 3 prélèvements sanguins sont réalisés afin de déterminer le niveau basal de glucose.

L'injection d'insuline est réalisée en sous-cutané au niveau du cou, sous l'oreille de l'animal à l'aide du stylo à insuline Novopen équipé d'une aiguille 31 G. Dans le cas de la double injection réalisée en contrôle, une injection est faite de chaque côté du cou.

Des prélèvements sanguins sont ensuite réalisés toutes les 10 minutes pendant 1 heure puis toutes les 30 minutes jusqu'à 2 heures, puis toutes les heures jusqu'à 16 heures. Après chaque prélèvement, le cathéter est rincé avec une solution diluée d'héparine.

Une goutte de sang est prélevée pour déterminer la glycémie au moyen d'un glucomètre.

Les courbes de pharmacodynamie du glucose sont ensuite tracées et comparées.

### Exemple D2 : Résultats de Pharmacodynamie des solutions d'insulines

| Exemples | | Insuline | Doses | Co-Polyamino acide | Nombre de cochons |
|---|---|---|---|---|---|
| B2 B4 | Double injection | lispro 100 UI/ml | 0,05 UI/kg | - | 10 |
| | | glargine 100 UI/ml | 0,15 UI/kg | | |
| B32 | Injection combinée | lispro+glargine (100 UI/ml/300UI/ml) | 0,2 UI/kg | 5 | 8 |

Les résultats de pharmacodynamie obtenus avec les formulations décrites dans les exemples B2 et B4 sont présentés figure 1. Selon l'invention, l'analyse de ces courbes montre (Figure 1) que la formulation combinée insuline glargine (300 IU/ml) insuline lispro (100 IU/ml) à pH 7 (triangles Δ) avec comme excipient le co-polyaminoacide 5 de l'exemple B32 se comporte de manière similaire à la double injection contrôle (carrés pleins ■) de formulation B2 et B4. La chute de glycémie dans les 30 premières minutes est similaire pour les deux formulations indiquant que la présence de co-polyaminoacide 5 ne perturbe pas le caractère rapide de l'insuline lispro. De même, les profils entre 30 min et 4h correspondant à la fin de l'insuline rapide et entre 4h et 16h correspondant à l'insuline basale sont similaires pour la double injection contrôle et pour la formulation. La combinaison des deux insulines lispro et glargine en présence du co-polyaminoacide 5 ne change donc pas le profil de ces deux insulines.

## Revendications

1. Composition sous forme d'une solution aqueuse injectable, dont le pH est compris entre 6,0 et 8,0, comprenant au moins :
a) une insuline basale dont le point isoélectrique pI est compris entre 5,8 et 8,5 ;
b) un co-polyaminoacide porteur de charges carboxylates et substitué par des groupements hydrophobes, choisi parmi les co-polyaminoacides de formule I : 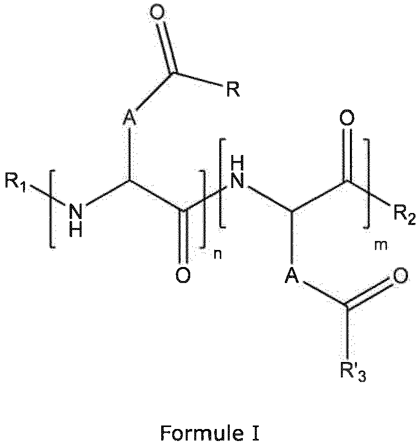 dans laquelle,
• A représente, indépendamment, soit un groupe -CH₂- (unité aspartique) soit un groupe -CH₂-CH₂- (unité glutamique),
• R₁ est un radical choisi dans le groupe constitué par un H, un groupe acyle linéaire en C2 à C10, un groupe acyle ramifié en C3 à C10, un benzyle, une unité « acide aminé » terminale et un pyroglutamate,
• R₂ est un radical -NR'R", R' et R" identiques ou différents étant choisis dans le groupe constitué par H, les alkyles linéaires ou ramifiés ou cycliques en C2 à C30, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S,
• R'₃ est un radical choisi dans le groupe constitué par les radicaux de formules -OR₃, II ou II': 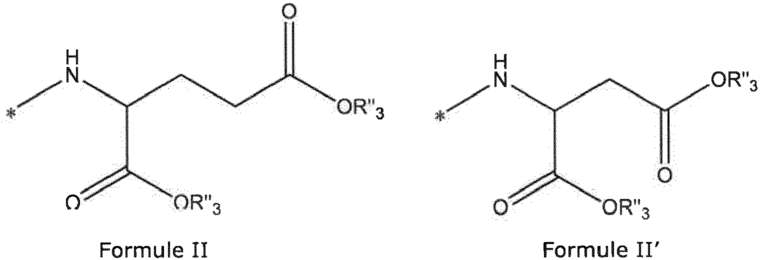 dans lesquelles * indique le site de rattachement au co-polyaminoacide
- R₃ et R"₃, identiques ou différents, représentent un H ou une entité cationique choisie dans le groupe comprenant les cations métalliques,
• -R est un radical choisi dans le groupe constitué par un radical en C8 à C30 linéaire ou ramifié, saturé ou insaturé, pouvant comporter des hétéroatomes ou un radical en C8 à C30 pouvant former des cycles carbonés ou pouvant comporter des hétéroatomes saturés, insaturés et/ou aromatiques, lesdits cycles pouvant être ortho-condensés ou péri-condensés, ou un radical de formule III ou III' tel que défini ci-dessous : 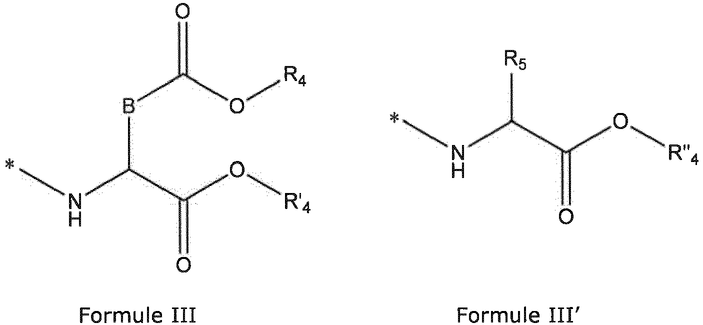 dans lesquelles, * indique le site de rattachement au co-polyaminoacide, et
- R₄ et R'₄, identiques ou différents représentent un H, une entité cationique choisie dans le groupe comprenant les cations métalliques, un radical R"₄ ou un radical R'''₄, et au moins un des R₄ et R'₄ est égal à R"₄.
∘ R"₄ représente un radical en C8 à C30 linéaire ou ramifié, saturé ou insaturé, pouvant comporter des hétéroatomes ou un radical en C8 à C30 pouvant former des cycles carbonés ou pouvant comporter des hétéroatomes saturés, insaturés et/ou aromatiques, lesdits cycles pouvant être ortho-condensés ou péri-condensés,
∘ R'''₄ représente un radical en C1 à C7 linéaire ou ramifié, saturé ou insaturé, pouvant comporter des hétéroatomes ou un radical en C1 à C7 pouvant former des cycles carbonés ou pouvant comporter des hétéroatomes saturés, insaturés et/ou aromatiques, lesdits cycles pouvant être ortho-condensés ou péri-condensés, et
- B représente, indépendamment soit un groupe -CH₂- (unité aspartique) soit un groupe -CH₂-CH₂- (unité glutamique),
- R₅ est un radical choisi dans le groupe constitué par un H, un alkyle linéaire ou ramifié en C1 à C4 ou un groupement benzyle,
• n/(n+m) est défini comme le taux de greffage molaire en radical hydrophobe des unités monomériques et est compris entre 1 et 50 % molaire,
• n + m représente le degré de polymérisation du co-polyaminoacide, c'est-à-dire le nombre moyen d'unités monomériques par chaîne de co-polyaminoacide et 5 ≤ n + m ≤ 1000.

2. Composition selon la revendication 1, **caractérisée en ce que** le co-polyaminoacide est choisi parmi les co-polyaminoacides de formule IV: 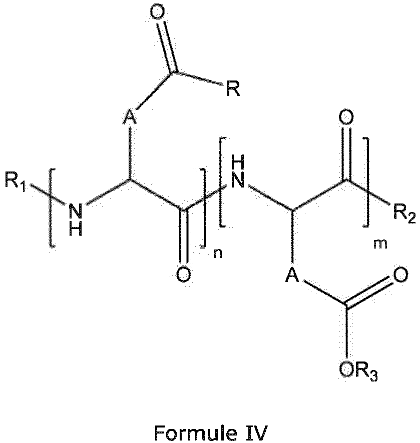 dans laquelle,
• A représente, indépendamment un groupe -CH₂- (unité aspartique) ou un groupe -CH₂-CH₂- (unité glutamique),
• R₁ est choisi dans le groupe constitué par un H, un groupe acyle linéaire en C2 à C10, un groupe acyle ramifié en C3 à C10, un benzyle, une unité « acide aminé » terminale et un pyroglutamate,
• R₂ est un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble des cycles carbonés saturés, insaturés et/ou aromatiques et/ou pouvant comporter des hétéroatomes, choisis dans le groupe constitué par O, N et S,
• les groupes R₃ identiques ou différents sont choisis dans le groupe constitué par un H ou une entité cationique choisie dans le groupe comprenant les cations métalliques,
• les groupes R représentent chacun indépendamment les uns des autres un radical choisi parmi les radicaux de formule générale V ou V' : 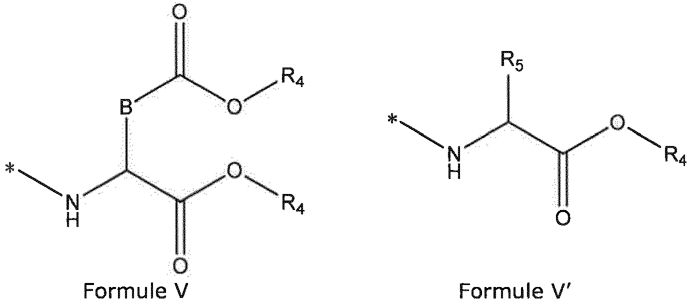 dans lesquelles, * indique le site de rattachement au co-polyaminoacide, et,
- R₄ représente un radical en C8 à C30 linéaire ou ramifié, saturé ou insaturé, pouvant comporter des hétéroatomes ou un radical en C8 à C30 pouvant former des cycles carbonés ou pouvant comporter des hétéroatomes saturés, insaturés et/ou aromatiques, lesdits cycles pouvant être ortho-condensés ou péri-condensés,
- B représente, indépendamment un groupe -CH₂- (unité aspartique) ou un groupe -CH₂-CH₂- (unité glutamique),
- R₅ représente indépendamment un H, un alkyle linéaire ou ramifié en C1 à C4 ou un groupement benzyle, ou
• R est un radical en C8 à C30 linéaire ou ramifié, saturé ou insaturé, pouvant comporter des hétéroatomes ou un radical en C8 à C30 pouvant former des cycles carbonés ou pouvant comporter des hétéroatomes saturés, insaturés et/ou aromatiques, lesdits cycles pouvant être ortho-condensés ou péri-condensés,
• n/(n+m) est défini comme le taux de greffage molaire en radical hydrophobe des unités monomériques et est compris entre 1 et 50 % molaire,
• n + m représente le degré de polymérisation du co-polyaminoacide, c'est-à-dire le nombre moyen d'unités monomériques par chaîne de co-polyaminoacide et 5 ≤ n + m ≤ 1000.

3. Composition selon les revendications 1 ou 2 **caractérisée en ce que** le co-polyaminoacide est choisi parmi les co-polyaminoacides de formule I ou IV, dans lesquelles le groupe A est un groupe -CH₂- (unité aspartique).

4. Composition selon la revendication 3, **caractérisée en ce que** les co-polyaminoacides de formule IV peuvent en outre comprendre des unités monomériques de formule VI" et/ou VI' :

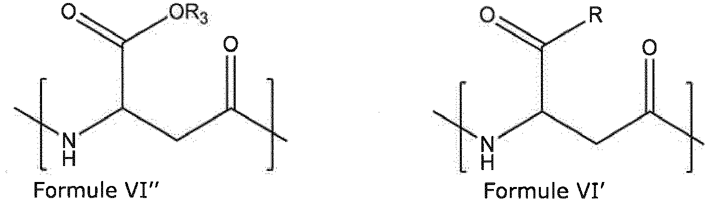

5. Composition selon les revendications 1 ou 2, **caractérisée en ce que** le co-polyaminoacide est choisi parmi les co-polyaminoacides de formules I ou IV, dans lesquelles le groupe A est un groupe -CH₂-CH₂- (unité glutamique).

6. Composition selon l'une quelconque des revendications précédentes, **caractérisée en ce que** le co-polyaminoacide est choisi parmi les co-polyaminoacides de formule I ou IV, dans laquelle R est choisi dans le groupe des radicaux issus des alcools hydrophobes.

7. Composition selon l'une quelconque des revendications 1 à 5, **caractérisée en ce que** le co-polyaminoacide est choisi parmi les co-polyaminoacides de formule I ou IV, dans laquelle R est un radical de type -L-R''', le radical R''' étant issu d'un acide hydrophobe et le radical L étant un radical trivalent comprenant de 2 à 10 carbones linéaire ou ramifié.

8. Composition selon l'une quelconque des revendications précédentes, **caractérisée en ce que** le co-polyaminoacide est choisi parmi les co-polyaminoacides de formules I ou IV, dans lesquelles R₄, R'₄ et/ou R"₄, identiques ou différents, sont choisis dans le groupe des radicaux issus des alcools hydrophobes.

9. Composition selon l'une quelconque des revendications précédentes, **caractérisée en ce que** l'insuline basale dont le point isoélectrique est compris entre 5,8 et 8,5 est l'insuline glargine

10. Composition selon l'une quelconque des revendications précédentes, **caractérisée en ce qu'**elle comprend entre 40 et 500 UI/mL d'insuline basale dont le point isoélectrique est compris entre 5,8 et 8,5.

11. Composition selon l'une quelconque des revendications précédentes, **caractérisée en ce que** la concentration en co-polyaminoacide substitué est au plus de 60 mg/mL.

12. Composition selon l'une quelconque des revendications précédentes, **caractérisée en ce qu'**elle comprend en outre une insuline prandiale.

13. Composition selon l'une quelconque des revendications 11 et 12, **caractérisée en ce que**, au total elle comprend entre 40 et 500 UI/mL d'insuline avec une combinaison d'insuline prandiale et d'insuline basale dont le point isoélectrique est compris entre 5,8 et 8,5.

14. Composition selon l'une quelconque des revendications précédentes, **caractérisée en ce qu'**elle comprend en outre une hormone gastrointestinale.

15. Formulation unidose à pH compris entre 7 et 7,8 comprenant une composition selon l'une quelconque des revendications précédentes.

## Patentansprüche

1. Zusammensetzung in Form einer injizierbaren wässrigen Lösung, deren pH zwischen 6,0 und 8,0 beträgt, wenigstens umfassend:
a) ein Grundinsulin, dessen isoelektrischer Punkt zwischen 5,8 und 8,5 beträgt;
b) eine Co-Polyaminosäure, die Carboxylat-Ladungen trägt und mit hydrophoben Gruppen substituiert ist, ausgewählt aus den Co-Polyaminosäuren der Formel I: 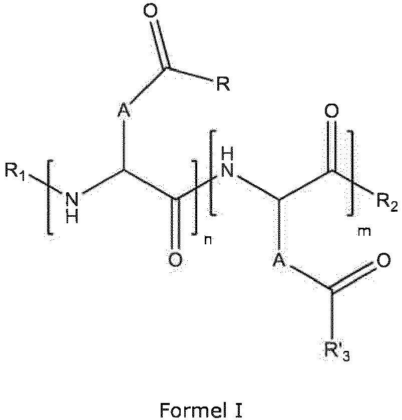 worin
• A, unabhängig, entweder eine -CH₂- -Gruppe (aspartische Einheit) oder eine -CH₂-CH₂- -Gruppe (glutamische Einheit) darstellt,
• R₁ ein Radikal ist, das ausgewählt ist aus der Gruppe bestehend aus einem H, einer linearen C2- bis C10-Acylgruppe, einer verzweigten C3-bis C10-Gruppe, einem Benzyl, einer terminalen « Aminosäure »-Einheit und einem Pyroglutamat,
• R₂ ein Radikal -NR'R" ist, wobei R' et R" identisch oder verschieden und ausgewählt sind aus der Gruppe bestehend aus H, linearen oder verzweigten oder zyklischen C2- bis C30-Alkylen und Benzyl, wobei die R'- und R"-Alkyle zusammen einen oder mehrere gesättigte, ungesättigte und/oder aromatische Kohlenstoffringe bilden und/oder Heteroatome tragen können, die ausgewählt sind aus der Gruppe bestehend aus O, N und S,
• R'₃ ein Radikal ist, das ausgewählt ist aus der Gruppe bestehend aus den Radikalen der Formeln -OR₃, II oder II': 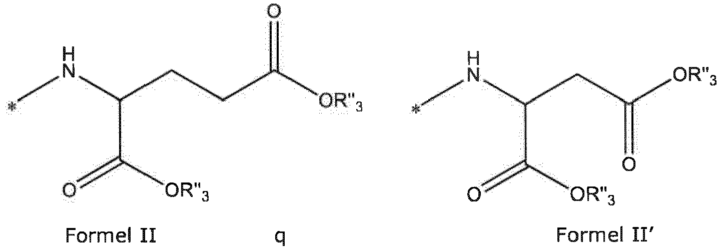 in denen * die Stelle der Bindung an die Co-Polyaminosäure anzeigt
- R₃ und R"₃, identisch oder verschieden sind und H oder eine kationische Einheit darstellen, die ausgewählt ist aus der Gruppe umfassend Metallkationen,
• -R ein Radikal ist, das ausgewählt ist aus der Gruppe bestehend aus einem linearen oder verzweigten, gesättigten oder ungesättigten C8- bis C30-Radikal, das Heteroatome tragen kann, oder einem C8- bis C30-Radikal, das gesättigte, ungesättigte und/oder aromatische Kohlenstoffringe bilden oder Heteroatome tragen kann, wobei die Ringe ortho-kondensiert oder peri-kondensiert sein können, oder einem Radikal der Formel III oder III' wie unten definiert : 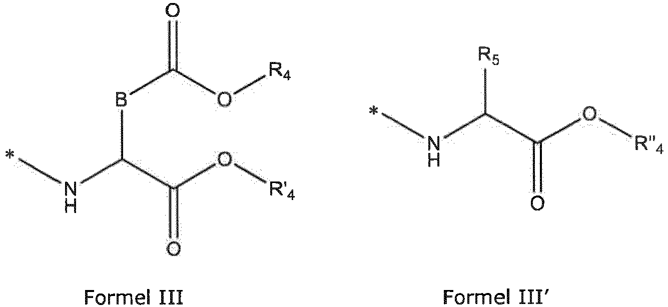 in denen * die Stelle der Bindung an die Polyaminosäure anzeigt, und
- R₄ und R'₄, die identisch oder verschieden sein können, ein H oder eine kationische Einheit darstellen, die ausgewählt ist aus der Gruppe umfassend Metallkationen, ein R"₄ -Radikal oder ein R'''₄ - Radikal, und wenigstens eines von R₄ und R'₄ ist identisch mit R"₄,
∘ R"₄ ein lineares oder verzweigtes, gesättigtes oder ungesättigtes C8- bis C30-Radikal darstellt, das Heteroatome tragen kann, oder ein C8- bis C30-Radikal, das gesättigte oder ungesättigte und/oder aromatische Kohlenstoffringe bilden oder Heteroatome tragen kann, wobei die Ringe ortho-kondensiert oder peri-kondensiert sein können,
∘ R'''₄ ein lineares oder verzweigtes, gesättigtes oder ungesättigtes C1- bis C7-Radikal darstellt, das Heteroatome tragen kann, oder ein C1- bis C7-Radikal, das gesättigte, ungesättigte und/oder aromatische Kohlenstoffringe bilden oder Heteroatome tragen kann, wobei die Ringe ortho-kondensiert oder peri-kondensiert sein können, und
- B, unabhängig, eine -CH₂- -Gruppe (aspartische Einheit) oder eine -CH₂-CH₂- -Gruppe (glutamische Einheit) darstellt,
- R₅ ein Radikal ist, das ausgewählt ist aus der Gruppe bestehend aus einem H, einem linearen oder verzweigten C1- bis C4-Alkyl oder einer Benzylgruppe,
• n/(n+m) definiert ist als das molare Ausmaß an Aufpropfen der monomeren Einheiten mit hydrophobem Radikal und zwischen 1 und 50 Mol-% beträgt,
• n + m den Polymerisationsgrad der Co-Polyaminosäure darstellt, d.h. die durchschnittliche Anzahl von monomeren Einheiten pro Kette der Co-Polyaminosäure, und 5 ≤ n + m ≤ 1000.

2. Zusammensetzung nach Anspruch 1, **dadurch gekennzeichnet, dass** die Co-Polyaminosäure ausgewählt ist aus den Co-Polyaminosäuren der Formel IV: 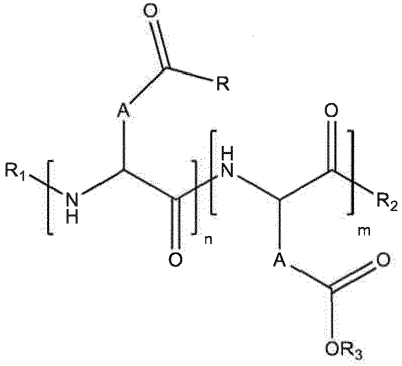 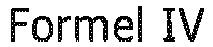 worin
• A, unabhängig, eine -CH₂- -Gruppe (aspartische Einheit) oder eine-CH₂-CH₂- -Gruppe darstellt (glutamische Einheit),
• R₁ ausgewählt ist aus der Gruppe bestehend aus einem H, einer linearen C2- bis C10-Acylgruppe, einer verzweigten C3- bis C10-Gruppe, einem Benzyl, einer terminalen « Aminosäure »-Einheit und einem Pyroglutamat,
• R₂ ein Radikal -NR'R" ist, wobei R' et R" identisch oder verschieden und ausgewählt sind aus der Gruppe bestehend aus H, linearen oder verzweigten oder zyklischen C2- bis C10-Alkylen und Benzyl, und wobei die R' und R" - Alkyle zusammen gesättigte, ungesättigte und/oder aromatische Kohlenstoffringe bilden und/oder die Heteroatome tragen können, die ausgewählt sind aus der Gruppe bestehend aus O, N und S,
• die R₃ -Gruppen, die identisch oder verschieden sind, ausgewählt sind aus der Gruppe bestehend aus einem H oder einer kationischen Einheit, die ausgewählt ist aus der Gruppe umfassend Metallkationen,
• die Gruppen R jeweils unabhängig voneinander ein Radikal darstellen, das ausgewählt ist aus den Radikalen der allgemeinen Formel V oder V': 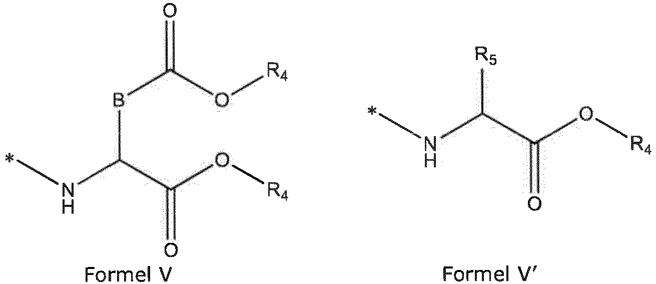 in denen * die Stelle der Bindung an die Co-Polyaminosäure anzeigt, und
- R₄ ein lineares oder verzweigtes, gesättigtes oder ungesättigtes C8- bis C30-Radikal darstellt, das Heteroatome tragen kann, oder ein C8- bis C30- Radikal, das gesättigte, ungesättigte und/oder aromatische Kohlenstoffringe bilden oder Heteroatome tragen kann, wobei die Ringe ortho-kondensiert oder peri-kondensiert sein können,
- B, unabhängig, eine -CH₂- -Gruppe (aspartische Einheit) oder eine -CH₂-CH₂- -Gruppe (glutamische Einheit) darstellt,
- R₅, unabhängig, ein H, ein lineares oder verzweigtes C1- bis C4-Alkyl oder eine Benzylgruppe darstellt, oder
• R ein lineares oder verzweigtes, gesättigtes oder ungesättigtes C8- bis C30-Radikal ist, das Heteroatome tragen kann, oder ein C8- bis C30-Radikal, das gesättigte, ungesättigte und /oder aromatische Kohlenstoffringe bilden oder Heteroatome tragen kann, wobei die Ringe ortho-kondensiert oder peri-kondensiert sein können,
• n/(n+m) definiert ist als das molare Ausmaß an Aufpropfen der monomeren Einheiten mit hydrophobem Radikal und zwischen 1 und 50 Mol-% beträgt,
• n + m den Polymerisationsgrad der Co-Polyaminosäure darstellt, d.h. die durchschnittliche Anzahl von monomeren Einheiten pro Kette der Co-Polyaminosäure, und 5 ≤ n + m ≤ 1000.

3. Zusammensetzung nach Anspruch 1 oder 2, **dadurch gekennzeichnet, dass** die Co-Polyaminosäure ausgewählt ist aus den Co-Polyaminosäuren der Formel I oder IV, in denen die Gruppe A eine -CH₂- -Gruppe (aspartische Einheit) ist.

4. Zusammensetzung nach Anspruch 3, **dadurch gekennzeichnet, dass** die Co-Polyaminosäuren der Formel IV unter anderem monomere Einheiten der Formel VI" und/oder VI' umfassen können:

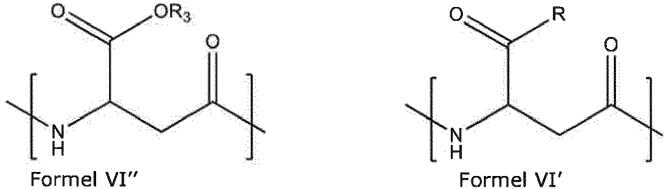

5. Zusammensetzung nach Anspruch 1 oder 2, **dadurch gekennzeichnet, dass** die Co-Polyaminosäure ausgewählt ist aus den Co-Polyaminosäuren der Formeln I oder IV, in denen die Gruppe A eine -CH₂-CH₂- -Gruppe (glutamische Einheit) ist.

6. Zusammensetzung nach einem der vorangehenden Ansprüche, **dadurch gekennzeichnet, dass** die Co-Polyaminosäure ausgewählt ist aus den Co-Polyaminosäuren der Formel I oder IV, worin R ausgewählt ist aus der Gruppe von von hydrophoben Alkoholen abgeleiteten Radikalen.

7. Zusammensetzung nach einem der Ansprüche 1 bis 5, **dadurch gekennzeichnet, dass** die Co-Polyaminosäure ausgewählt ist aus den Co-Polyaminosäuren der Formel I oder IV, worin R ein Radikal vom Typ -L-R''' ist, wobei das Radikal R'" abgeleitet ist von einer hydrophoben Säure und das Radikal L ein trivalentes lineares oder verzweigtes Radikal ist, das 2 bis 10 Kohlenstoffe umfasst.

8. Zusammensetzung nach einem der vorangehenden Ansprüche, **dadurch gekennzeichnet, dass** die Co-Polyaminosäure ausgewählt ist aus den Co-Polyaminosäuren der Formeln I und IV, in denen R₄, R'₄ und/oder R"₄, identisch oder verschieden sind und ausgewählt sind aus der Gruppe von Radikalen, die von hydrophoben Alkoholen abgeleitet sind.

9. Zusammensetzung nach einem der vorangehenden Ansprüche, **dadurch gekennzeichnet, dass** das Grundinsulin, dessen isoelektrischer Punkt zwischen 5,8 und 8,5 beträgt, Insulin glargin ist.

10. Zusammensetzung nach einem der vorangehenden Ansprüche, **dadurch gekennzeichnet dass** sie zwischen 40 und 500 IU/ml Grundinsulin umfasst, dessen isoelektrischer Punkt zwischen 5,8 und 8,5 beträgt.

11. Zusammensetzung nach einem der vorangehenden Ansprüche, **dadurch gekennzeichnet, dass** die Konzentration der substituierten Co-Polyaminosäure höchstens 60 mg/mL beträgt.

12. Zusammensetzung nach einem der vorangehenden Ansprüche, **dadurch gekennzeichnet, dass** sie unter anderem ein prandiales Insulin umfasst.

13. Zusammensetzung nach einem der Ansprüche 11 und 12, **dadurch gekennzeichnet, dass** sie insgesamt zwischen 40 und 500 IU/mL Insulin mit einer Kombination aus prandialem Insulin und Grundinsulin umfasst, dessen isoelektrischer Punkt zwischen 5,8 und 8,5 beträgt.

14. Zusammensetzung nach einem der vorangehenden Ansprüche, **dadurch gekennzeichnet, dass** sie unter anderem ein gastrointestinales Hormon umfasst.

15. Einzeldosisformulierung mit einem pH zwischen 7 und 7,8, umfassend eine Zusammensetzung nach einem der vorangehenden Ansprüche.

## Claims

1. Composition in the form of an injectable aqueous solution, whose pH is comprised from 6.0 to 8.0, comprising at least:
a) a basal insulin, whose isoelectric point pI is comprised from 5.8 to 8.5;
b) a co-polyamino acid bearing carboxylate charges and substituted with hydrophobic groups, chosen from the co-polyamino acids of formula I: 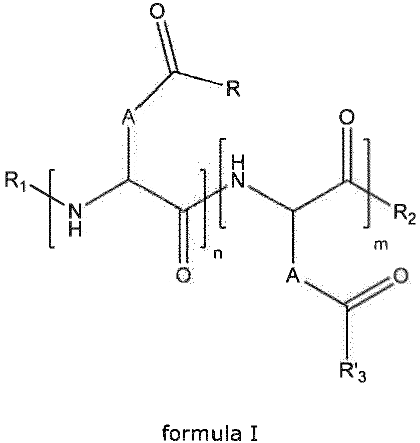 in which:
• A independently represents either a -CH₂- group (aspartic unit) or a -CH₂-CH₂- group (glutamic unit),
• R₁ is a radical chosen from the group consisting of an H, a linear C₂ to C₁₀ acyl group, a branched C₃ to C₁₀ acyl group, a benzyl, a terminal "amino acid" unit and a pyroglutamate,
• R₂ is an -NR'R" radical, R' and R", which may be identical or different, being chosen from the group consisting of H, linear or branched or cyclic C₂ to C₃₀ alkyls and benzyl, and said R' and R" alkyls being alkyls which can together form one or more saturated, unsaturated and/or aromatic rings which are carbon-based and/or which can comprise heteroatoms, chosen from the group consisting of O, N and S,
• R'₃ is a radical chosen from the group consisting of the radicals of formulae -OR₃, II or II': 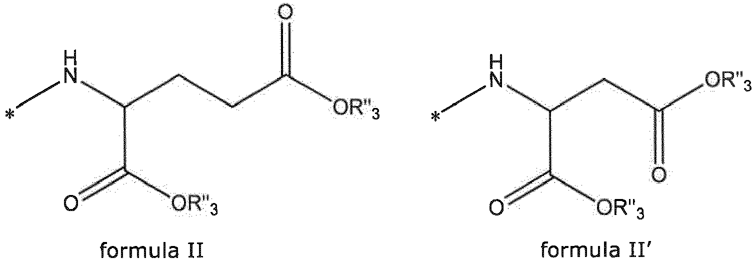 in which * indicates the site of attachment of the co-polyamino acid
- R₃ and R"₃, which may be identical or different, represent an H or a cationic entity chosen from the group comprising metal cations,
• -R is a radical chosen from the group consisting of a saturated or unsaturated, linear or branched C₈ to C₃₀ radical which can comprise heteroatoms or a C₈ to C₃₀ radical which can form rings which are carbon-based or which can comprise heteroatoms, which are saturated, unsaturated and/or aromatic, said rings possibly being ortho-condensed or peri-condensed, or a radical of formula III or III' as defined below: 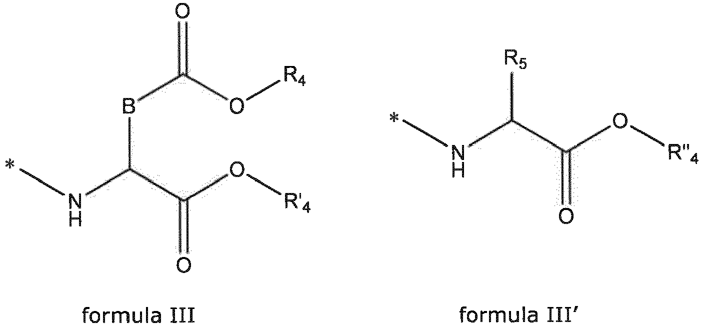 In which * Indicates the site of attachment to the co-polyamino acid, and
- R₄ and R'₄, which may be identical or different, represent an H, a cationic entity chosen from the group comprising metal cations, an R"₄ radical or an R'''₄ radical, and at least one of R₄ and R'₄ is equal to R"₄.
∘ R"₁ represents a saturated or unsaturated, linear or branched C₈ to C₃₀ radical which can comprise heteroatoms or a C₈ to C₃₀ radical which can form rings which are carbon-based or which can comprise heteroatoms, which are saturated, unsaturated and/or aromatic, said rings possibly being ortho-condensed or peri-condensed,
∘ R'''₄ represents a saturated or unsaturated, linear or branched C₁ to C₇ radical which can comprise heteroatoms or a C₁ to C₇ radical which can form rings which are carbon-based or which can comprise heteroatoms, which are saturated, unsaturated and/or aromatic, said rings possibly being ortho-condensed or peri-condensed, and
- B independently represents either a -CH₂- group (aspartic unit) or a -CH₂-CH₂- group (glutamic unit),
- R₅ is a radical chosen from the group consisting of an H, a linear or branched C₁ to C₄ alkyl or a benzyl group,
• n/(n+m) is defined as the molar degree of grafting with hydrophobic radical of the monomeric units and is comprised from 1 to 50 mol%,
• n + m represents the degree of polymerization of the co-polyamino acid, i.e. the average number of monomeric units per chain of co-polyamino acid, and 5 ≤ n + m ≤ 1000.

2. The composition as claimed in claim 1, wherein the co-polyamino acid is chosen from the co-polyamino acids of formula IV: 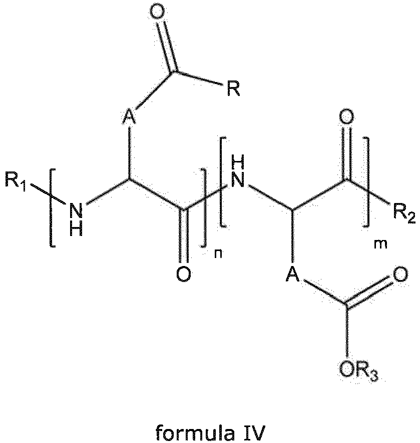 in which:
• A independently represents a -CH₂- group (aspartic unit) or a -CH₂-CH₂- group (glutamic unit),
• R₁ is chosen from the group consisting of an H, a linear C₂ to C₁₀ acyl group, a branched C₃ to C₁₀ acyl group, a benzyl, a terminal "amino acid" unit and a pyroglutamate,
• R₂ is an -NR'R" radical, R' and R", which may be identical or different, being chosen from the group consisting of H, linear or branched or cyclic C₂ to C₁₀ alkyls and benzyl, and said R' and R" alkyls being alkyls which can together form saturated, unsaturated and/or aromatic rings which are carbon-based and/or which can comprise heteroatoms, chosen from the group consisting of O, N and S,
• the R₃ groups, which may be identical or different, are chosen from the group consisting of an H or a cationic entity chosen from the group comprising metal cations,
• the R groups each represent, independently from one another, a radical chosen from the radicals of general formula V or V': 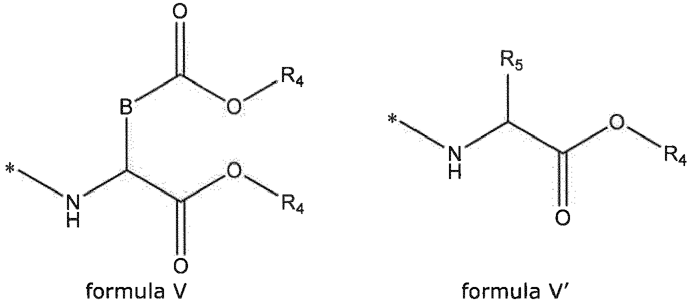 in which * indicates the site of attachment to the co-polyamino acid, and
- R₄ represents a saturated or unsaturated, linear or branched C₈ to C₃₀ radical which can comprise heteroatoms or a C₈ to C₃₀ radical which can form rings which are carbon-based or which can comprise heteroatoms, which are saturated, unsaturated and/or aromatic, said rings possibly being ortho-condensed or peri-condensed,
- B independently represents a -CH₂- group (aspartic unit) or a -CH₂-CH₂- group (glutamic unit),
- R₅ independently represents an H, a linear or branched C₁ to C₄ alkyl or a benzyl group, or
• R is a saturated or unsaturated, linear or branched C₈ to C₃₀ radical which can comprise heteroatoms or a C₈ to C₃₀ radical which can form rings which are carbon-based or which can comprise heteroatoms, which are saturated, unsaturated and/or aromatic, said rings possibly being ortho-condensed or peri-condensed,
• n/(n+m) is defined as the molar degree of grafting with hydrophobic radical of the monomeric units, and is comprised from 1 to 50 mol%,
• n + m represents the degree of polymerization of the co-polyamino acid, i.e. the average number of monomeric units per chain of co-polyamino acid, and 5 ≤ n + m ≤ 1000.

3. The composition as claimed in claim 1 or 2, wherein the co-polyamino acid is chosen from the co-polyamino acids of formula I or IV, in which the group A is a -CH₂- group (aspartic unit).

4. The composition as claimed in claim 3, wherein the co-polyamino acids of formula IV can also comprise monomeric units of formula VI" and/or VI':

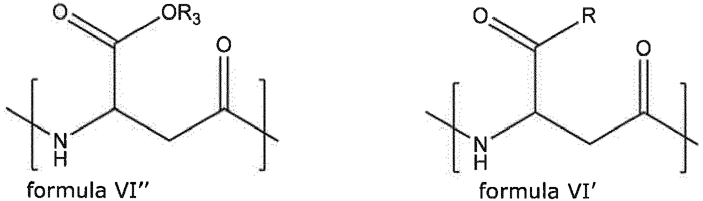

5. The composition as claimed in claim 1 or 2, wherein the co-polyamino acid is chosen from the co-polyamino acids of formula I or IV, in which the group A is a -CH₂-CH₂- group (glutamic unit).

6. The composition as claimed in any one of the preceding claims, wherein the co-polyamino acid is chosen from the co-polyamino acids of formula I or IV, in which R is chosen from the group of radicals derived from hydrophobic alcohols.

7. The composition as claimed in either one of claims 1 to 5, wherein the co-polyamino acid is chosen from the co-polyamino acids of formula I or IV, in which R is a radical of -L-R''' type, the R'" radical being derived from a hydrophobic acid and the L radical being a linear or branched trivalent radical comprising from 2 to 10 carbons.

8. The composition as claimed in any one of the preceding claims, wherein the co-polyamino acid is chosen from the co-polyamino acids of formula I or IV, in which R₄, R'₄ and/or R"₄, which may be identical or different, are chosen from the group of the radicals derived from hydrophobic alcohols.

9. The composition as claimed in any one of the preceding claims, wherein the basal insulin, whose isoelectric point is comprised from 5.8 to 8.5, is insulin glargine.

10. The composition as claimed in any one of the preceding claims, which comprises from 40 to 500 IU/ml of basal insulin, whose isoelectric point is comprised from 5.8 to 8.5.

11. The composition as claimed in any one of the preceding claims, wherein the concentration of substituted co-polyamino acid is at most 60 mg/ml.

12. The composition as claimed in any one of the preceding claims, which also comprises a prandial insulin.

13. The composition as claimed in either one of claims 11 and 12, which comprises in total from 40 to 500 IU/ml of insulin with a combination of prandial insulin and basal insulin, whose isoelectric point is comprised from 5.8 to 8.5.

14. The composition as claimed in any one of the preceding claims, which also comprises a gut hormone.

15. A single-dose formulation at a pH comprised from 7 to 7.8, comprising a composition as claimed in any one of the preceding claims.
